# FASCICULE DE BREVET EUROPEEN

(11) **EP 0 581 677 B1**
(45) Date de publication et mention de la délivrance du brevet: **08.01.1997**
(21) Numéro de dépôt: 93401959.7
(22) Date de dépôt: 28.07.1993
(51) Int. Cl.: C07C 225/18, A61K 31/13, C07C 251/44, C07D 311/22, A61K 31/35

(54) **Cycloalkylalkylamines ligands aux récepteurs sigma, leur procédé de préparation et leur application thérapeutique**
Cycloalkylalkylamine als Liganden für sigma-Rezeptoren, Verfahren und ihre Herstellung und ihre Anwendung in der Therapie
Cycloalkylalkylamines which are sigma-receptor ligands, process for preparing them and their application in therapy

(30) Priorité: 31.07.1992 FR 9209536
(43) Date de publication de la demande: 02.02.1994
(73) Titulaire: INSTITUT DE RECHERCHE JOUVEINAL (I.R.J), F-94265 Fresnes Cedex (FR)
(72) Inventeur: Junien, Jean-Louis, F-92310 Sevres (FR); Calvet, Alain, F-94240 l'Hay-les-Roses (FR); Jacobelli, Henri, F-91550 Paray-Vieille-Poste (FR); Roman, François, F-94400 Vitry sur Seine (FR)

(56) Documents cités:
- EP-A- 0 383 318
- WO-A-91/09594

## Description

La présente invention a pour objet de nouvelles cycloalkylalkylamines, ligands aux récepteurs sigma, leur procédé de préparation et leur application en thérapeutique.

Depuis la mise en évidence des récepteurs sigma, les nombreux travaux effectués sur ce sujet ont montré leur implication dans divers dysfonctionnements psychiques et, plus récemment, leur implication locale dans certains désordres gastrointestinaux.

En conséquence, depuis quelques années, on a proposé de nombreuses molécules de structures chimiques diverses qui visent à posséder une affinité aux récepteurs sigma et pour lesquelles on a envisagé leur utilisation aux traitements des psychoses et/ou des désordres gastrointestinaux.

En fait la plupart de ces composés ne sont pas des ligands spécifiques aux récepteurs sigma et interagissent avec d'autres récepteurs parmi lesquels les récepteurs de la phencyclidine (PCP) et les récepteurs dopaminergiques (D₂); du fait de ces affinités multiples, ils ne peuvent être utilisés pour les applications thérapeutiques envisagées, sans risquer d'entrainer des effets secondaires graves, comme des manifestations extrapyramidales qui ne sont que difficilement ou partiellement réversibles.

Les plus connues de ces molécules appartiennent à des familles chimiquement disparates et sont entre autres :
- la N-allyl-normétazocine (SKF 10,047) et la cyclazocine qui ont une structure de type benzomorphane et qui, outre leur affinité sigma, ont une forte affinité pour les récepteurs de la phencyclidine (PCP), ceux-ci étant impliqués dans des manifestations psychotiques se traduisant par des états de désorientation, d'excitation ou d'hallucinations;
- la 1,3-di-o-tolylguanidine (DTG) dont la séquence chimique caractéristique est une guanidine et qui, outre sa difficulté à franchir la barrière hémato-méningée, provoque chez le rat des symptomes analogues à ceux de la PCP;
- la (+)-(3-hydroxyphényl)-3-N-(propyl-1)pipéridine (+)-3-PPP qui montre une forte affinité pour les récepteurs dopaminergiques (D₂);
- l'halopéridol (DCI) qui est la 4-[4-(p-chlorophényl)-4-hydroxypipéridino]-4'-fluorobutyrophénone utilisée pour ses propriétés neuroleptiques et qui est encore considéré comme composé ligand sigma de référence bien que son emploi s'avère particulièrement délicat de par sa forte affinité aux récepteurs dopaminergiques D₂ qui peut induire des troubles extrapyramidaux de type cataleptique.

Surmontant les difficultés de l'état de la technique il vient d'être découvert de nouveaux composés qui présentent une affinité remarquable pour les récepteurs sigma tout en étant sensiblement dépourvus de ces affinités indésirables précédemment énoncées. Cette activité sigma est concrétisée "in vivo" chez l'animal par l'aptitude des composés à inhiber les convulsions provoquées par électrochoc et également à inhiber les ulcères provoqués par la cystéamine, ce qui autorise leur utilisation comme thérapie de certains désordres du système nerveux central et/ou du tractus gastrointestinal.

La présente invention concerne donc des composés nouveaux qui sont des cycloalkylalkylamines ligands aux récepteurs sigma et de formule générale (I) 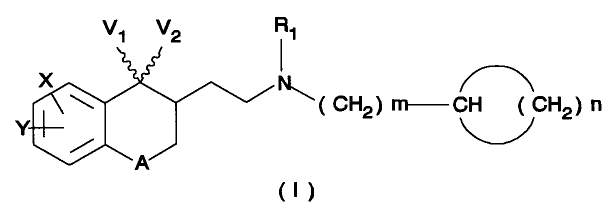 dans laquelle :
R₁ est H ou alkyl de C₁ à C₄ ;
X et Y identiques ou différents sont H, OH, alkyl de C₁ à C₄, alkoxy C₁ à C₄, halogène ou nitrile;
V₁ et V₂ forment ensemble une double liaison liée à un atome d'oxygène ou bien à un radical hydroxyimino N-OH, ou bien sont reliés en une chaîne éthylène dioxy -O-CH₂-CH₂-O-;
A représente une liaison de valence, un atome d'oxygène, un groupe méthylène ou encore un groupe éthylène;
m est égal à 0, 1 ou 2;
n a pour valeur un entier de 1 à 5.

L'invention se rapporte à la fois aux formes racémiques ou optiquement actives de ces composés (I) et à leurs sels d'addition avec des acides pharmaceutiquement acceptables. A cet effet on utilise à titre d'exemple, les sels avec les acides acétique, benzènesulfonique, camphosulfonique, citrique, éthanesulfonique, fumarique, bromhydrique, lactique, maléique, malique, méthanesulfonique, mucique, nitrique, pamoïque, phosphorique, salicylique, stéarique, succinique, sulfurique, tartrique et plus particulièrement l'acide chlorhydrique. Parmi les significations précédemment présentées, halogène signifie un atome de brome, de chlore ou encore de fluor qui est préféré.

Dans l'ensemble de ces composés on préfère ceux dans lesquels V₁ et V₂ forment ensemble une double liaison liée à un atome d'oxygène, A est égal à CH₂ pour former ainsi un cycle à 6 atomes de carbones, R₁ est alkyl de C₁ à C₄ et qui sont compris dans la formule (I.a)

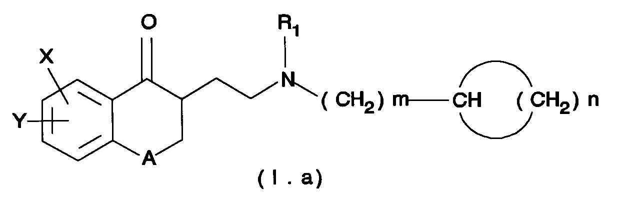

On préfère particulièrement les composés (I.a) dans lesquels R₁ est CH₃, m = 1 ou 2 et n = 2 ou 3.

Tel qu'annoncé, les études pharmacologiques des composés de l'invention montrent une affinité sigma surprenante et imprévisible au regard de ce qu'enseigne l'art antérieur.

Au brevet des Etats-Unis n° 3,189,612 il est décrit des structures dérivées d'indanone, intermédiaires de synthèse pour la préparation des composés visés par l'invention, composés antihistaminiques de formule (A) 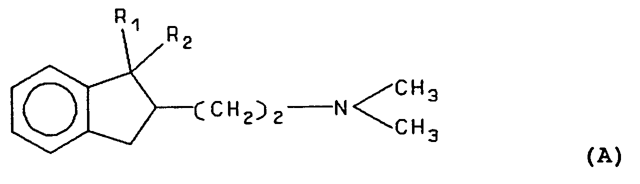
dans laquelle essentiellement R₁ et R₂ sont alkyl inférieurs.

Il n'est mentionné aucune activité pharmacologique pour les composés d'indanone dans lesquels R₁ et R₂ forment ensemble une fonction cétone.

Au brevet des Etats-Unis n°4,564,641 sont décrits des dérivés de tétrahydronaphtalènes de formule (B) 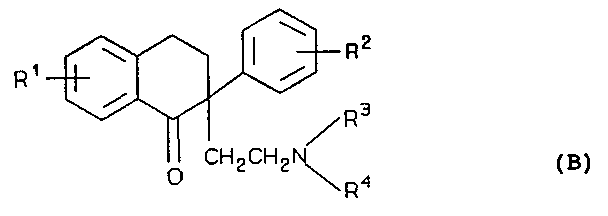 dans laquelle :
R₁ et R₂ sont identiques ou différents et sont chacun hydrogène, halogène, trifluorométhyl, C₁-C₄ alkyl ou C₁₋₄ alkoxy, R₃ est C₁-C₆ alkyl et R₄ est l'hydrogène, C₁-C₆ alkyl ou benzyl ou encore, R₃ et R₄ ensemble peuvent former une chaîne alkyl C₂-C₅.

Les composés décrits dans ce brevet sont différents de ceux de la présente invention notamment de par leur structure chimique qui est caractérisée par le fait que le carbone adjacent à la fonction carbonyle est substitué à la fois par un radical phényl et par un radical aminoéthyl N mono ou N disubstitué; d'autre part ces composés sont annoncés montrer une activité inhibitrice de la recapture de la noradrénaline. En aucun cas les divers articles de pharmacologie publiés postérieurement au brevet n'ont annoncé une affinité de ces composés aux récepteurs sigma.

Dans l'article paru dans J.Med.Chem., 1977, vol.20, n°5, 699-705, il est plus particulièrement décrit des composés de formule générale (C) 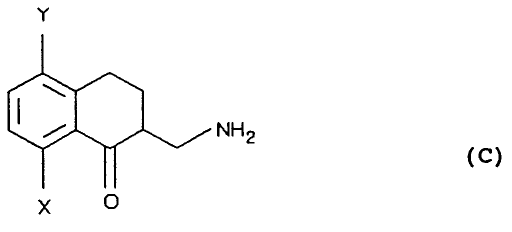
qui possèdent une activité analgésique et tranquilisante. Leur structure chimique est différente de celle des nouveaux composés de l'invention; ainsi, leur chaîne carbonée reliant le cycle tétralone à l'atome d'azote ne comporte qu'un seul atome de carbone et par ailleurs l'atome d'azote n'est en aucun cas substitué par un radical cycloalkylalkyl quelconque.

A la demande de brevet européen n°0,383,318 sont décrits des aralkylamines de formule (D) 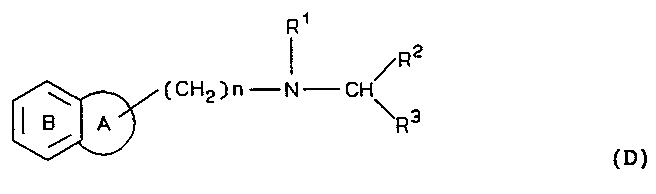 dans laquelle :
R₁ est hydrogène ou alkyl inférieur; R₂ est un groupe aromatique pouvant être substitué; R₃ est l'hydrogène, un groupe alkyl inférieur ou un groupe aromatique pouvant être substitué; n peut prendre des valeurs de 0 à 7; A est un cycle de 5 à 8 atomes de carbones éventuellement substitué, pouvant comprendre un ou deux hétéroatomes qui sont l'oxygène et le soufre; et B est un cycle benzène qui peut être substitué, et les sels physiologiquement acceptables de ces composés, qui sont utilisés comme inhibiteurs de la cholinestérase et comme agents améliorants la fonction cérébrale.

Les composés de cette demande européenne sont différents des nouveaux composés selon l'invention de formule (I) de par leur structure chimique, notamment par la nature des substituants de la fonction amine qui, dans la demande 0,383,318, comprend expressement un groupe aromatique R₂ qui est absent des structures de la présente invention, et, par ailleurs, de par la chaîne carbonée reliant le cycle A à la fonction amine qui peut comprendre de 0 à 7 atomes de carbone alors que dans la présente invention cette chaîne est exclusivement composée de deux atomes de carbone.

Egalement, les composés de la demande 0,383,318 diffèrent de la présente invention par leur propriété inhibitrice de la cholinestérase sans qu'ils soient décrits comme ligands aux récepteurs sigma, ni actifs sur le tractus gastrointestinal.

Un autre aspect de la présente invention est de viser un procédé de préparation des composés de formule (I) et de leurs sels. Essentiellement le procédé de préparation d'un composé (I) de formule (I.a) dans laquelle X, Y, A, R₁, m et n ont les significations précédemment définies, consiste tel que montré au schéma 1 :
i) à cycliser et décarboxyler par chauffage en milieu acide un intermédiaire malonique (VIII) 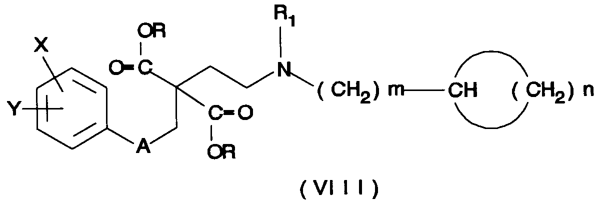 dans lequel R est l'hydrogène, alkyl de C₁ à C₄ ou cycloalkyl inférieur, ou
ii) à cycliser un intermédiaire acide (IX) 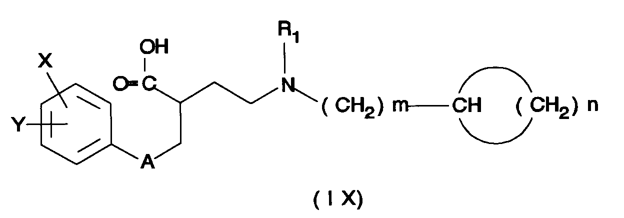 par une méthode d'acylation de type Friedel-Crafts, ou 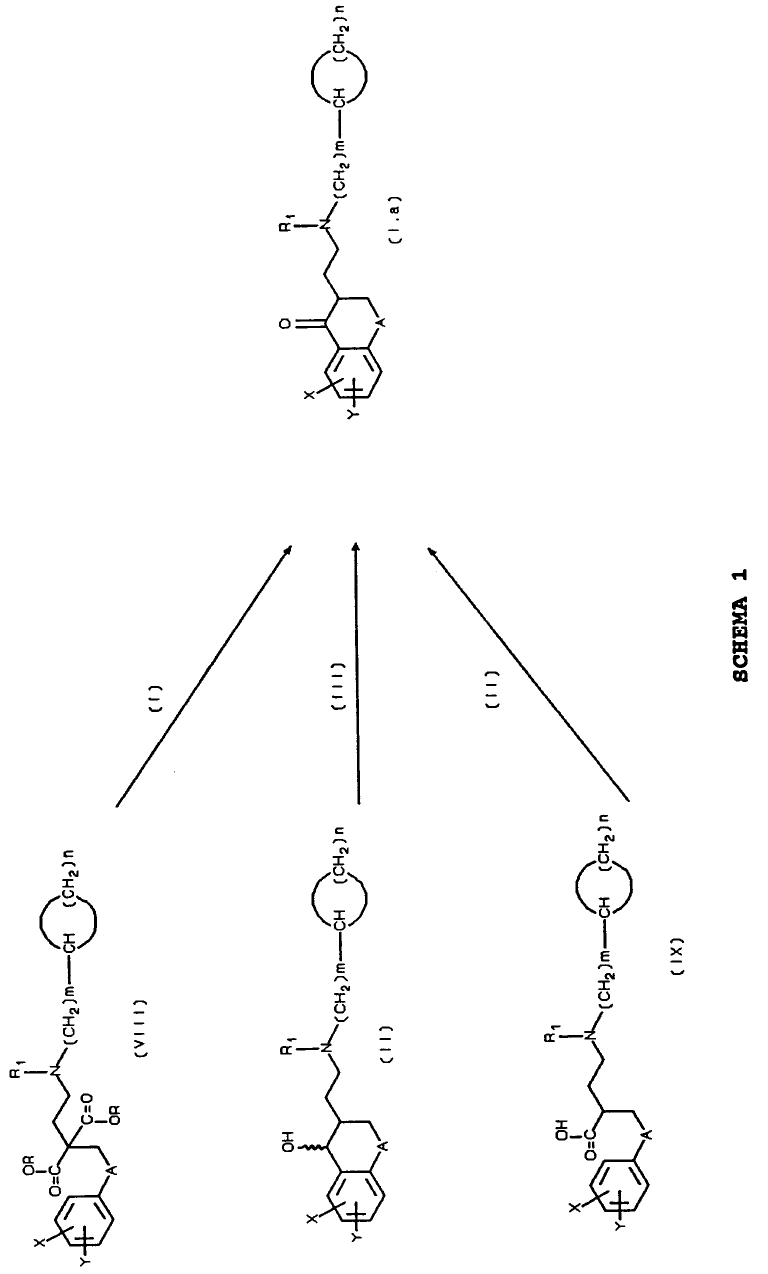
iii) selon le procédé préféré à procéder à l'oxydation de la fonction hydroxyle d'un amino alcool (II)

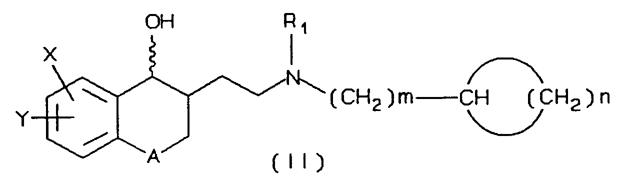

par un réactif oxydant approprié, et,
comme il est montré au schéma 2, 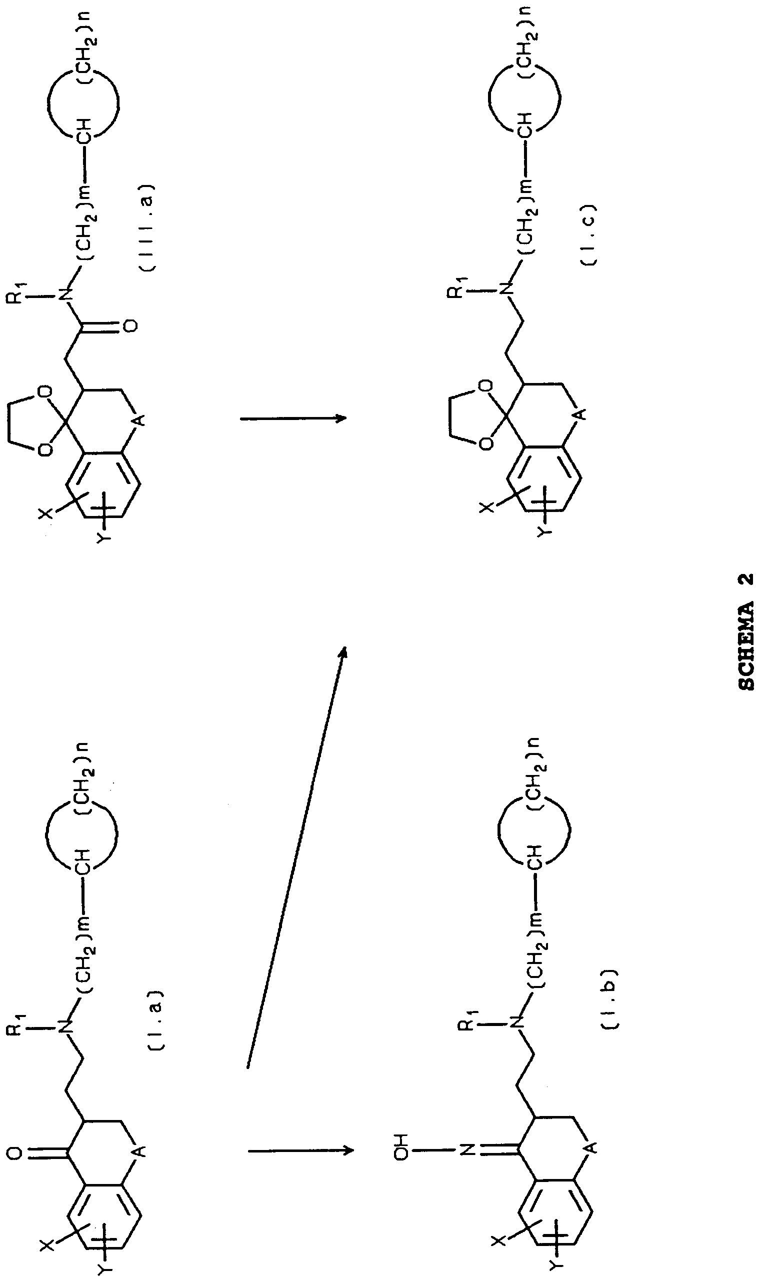
pour préparer un composé de l'invention (I) dans lequel V₁ et V₂ forment une double liaison liée à un radical hydroxyimino =N-OH, et qui répond à la formule (I.b) 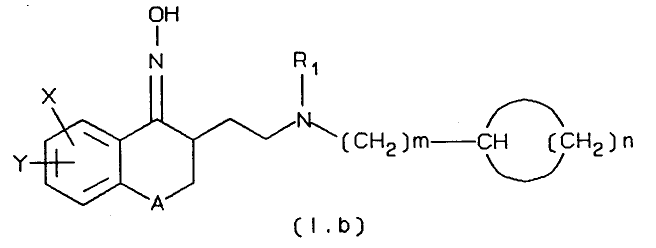
à faire réagir un composé (I.a) avec l'hydroxylamine et, pour préparer un composé de l'invention (I) dans lequel V₁ et V₂ sont reliés en une chaîne éthylène dioxy -O-CH₂-CH₂-O-, et qui répond à la formule (I.c) 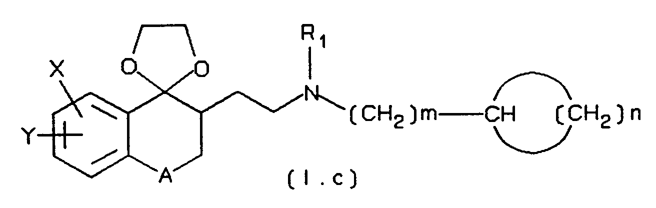
à faire réagir un composé (I.a) par condensation avec l'éthylène glycol éventuellement en présence d'un catalyseur ou, selon la méthode préférée, à réduire par un hydrure métallique ou organo métallique un intermédiaire amide (III.a) de formule

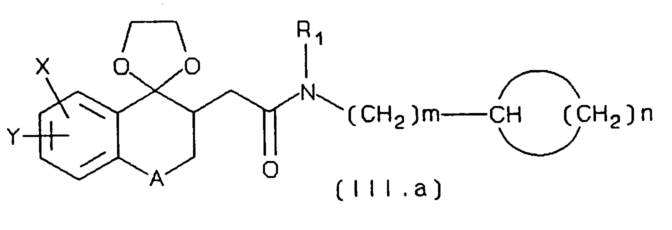

Tels qu'énoncés les procédés de préparation font appel aux intermédiaires (II), (III.a), (VIII) et (IX) dont la préparation est rapportée dans la suite de ce mémoire.

De façon générale le premier procédé, tel que montré au schéma 1 consiste à cycliser puis décarboxyler par chauffage en milieu acide un intermédiaire malonique (VIII) afin d'obtenir le composé (I.a). De tels intermédiaires (VIII) utilisés dans cette réaction sont connus et peuvent être notamment préparés selon la méthode décrite dans le brevet des Etats-Unis n° 3,189,612.

Le deuxième procédé, également illustré au schéma 1, consiste à cycliser un intermédiaire acide (IX), en présence d'un catalyseur et selon par exemple la réaction d'acylation dite de Friedel-Crafts, afin d'obtenir le composé (I.a). De tels intermédiaires (IX) utilisés dans cette réaction sont connus et peuvent notamment être préparés selon la méthode décrite dans le brevet des Etats-Unis n° 4,564,641.

Le troisième procédé, qui est celui préféré, met en jeu une réaction d'oxydation sur un amino alcool intermédiaire (II) pour obtenir le composé (I.a). Plus précisémment, la mise en oeuvre de ce procédé de préparation préféré présenté en iii) au schéma 1 consiste :
à oxyder la fonction hydroxyle de l'amino alcool secondaire (II) par un réactif approprié choisi dans le groupe de ceux proposés pour réaliser ces oxydations, qui sont décrits par exemple dans "Advanced Organic Chemistry" M. MARCH, 3^{d} Edition, p1057-1060. On utilise favorablement des dérivés du manganèse comme le permanganate de potassium (KMnO₄), l'oxyde de manganèse (MnO₂), ou des dérivés du chrome comme le trioxyde de chrome (CrO₃), le complexe CrO₃-pyridine, le dichromate de pyridinium et le chlorochromate de pyridinium qui est le réactif préféré. Avec ce réactif, la réaction est effectuée dans un solvant inerte qui peut être choisi parmi les solvants éthérés comme le diéthyléther, le méthyl-t.butyl éther, les éthers di-isopropyliques ou dibutyliques, le tétrahydrofurane (THF), le dioxane-1,4, ou bien le nitrobenzène, la pyridine, ou encore les hydrocarbures halogénés comprenant 1 à 6 atomes de carbones et parmi lesquels on préfère le chlorure de méthylène, et consiste de la façon qui est préférée, pour 1 mole de composé (II) à faire réagir 1,5 à 4 moles de chlorochromate de pyridinium à une température comprise entre 15 et 100°C selon le solvant et ce durant 15 à 30 heures. Plus précisément, la méthode consiste à ajouter à une température comprise entre 20 et 35°C une mole de composé (II), en solution dans le chlorure de méthylène, à 2,4 à 2,8 moles du réactif oxydant. A cette température, la réaction est habituellement complète après 20 à 25 heures et le composé de l'invention (I.a) résultant de l'oxydation est isolé et purifié par les méthodes usuelles qui sont décrites dans la partie expérimentale.

La préparation du composé intermédiaire (II) qui est le précurseur direct mis en oeuvre dans ce procédé préféré est illustrée au schéma 3 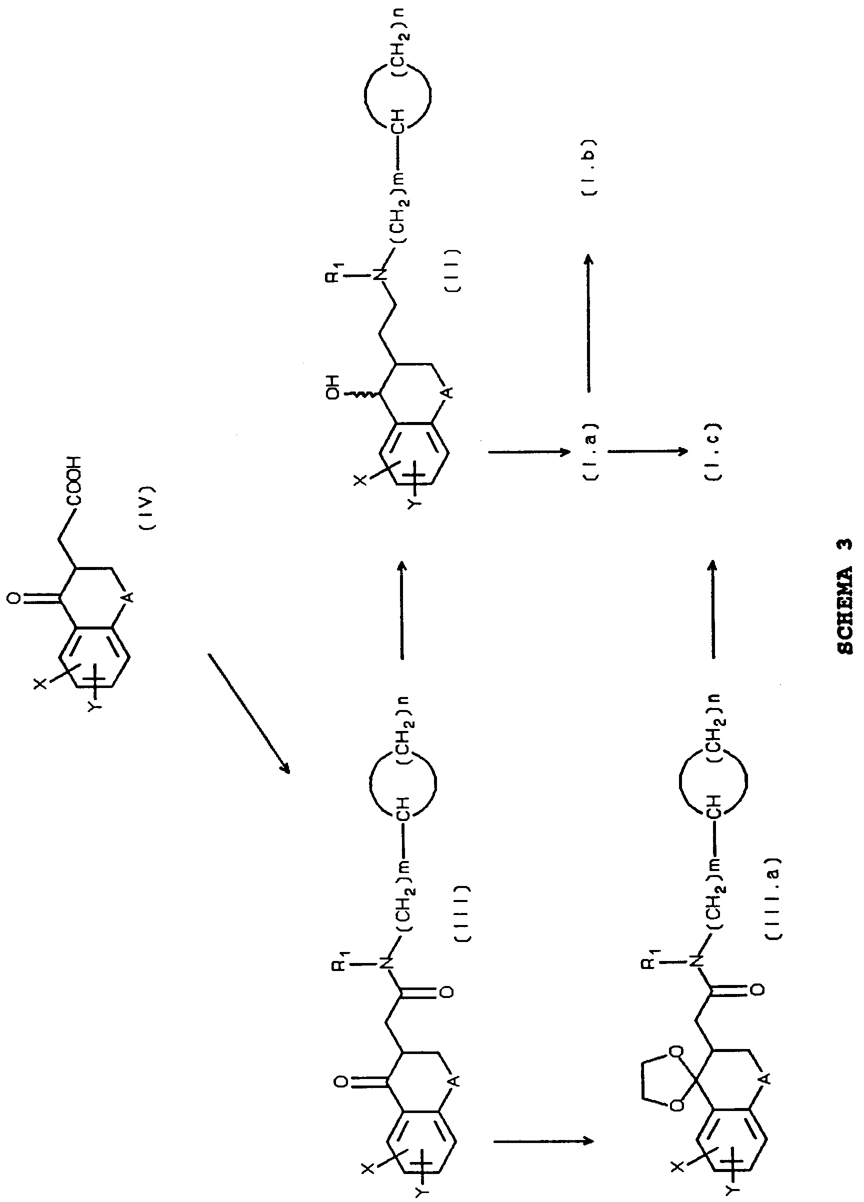 et consiste à partir d'un acide (IV), à en préparer l'amide (III) qui est ensuite réduit pour obtenir l'alcool (II).

D'une façon générale, les amides (III) qui sont des produits nouveaux, sont obtenus par addition d'une amine (XI) de formule 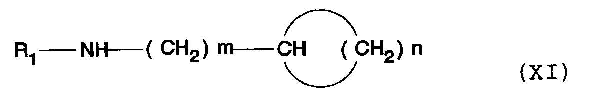 dans laquelle R₁ est l'hydrogène ou alkyl de C₁ à C₄,
sur un chlorure d'acide préparé à partir de l'acide précurseur (IV) et de chlorure de thionyle et dans les conditions de l'invention, on utilise généralement l'amine en solution dans un solvant inerte à l'agent réducteur utilisé, le chlorure de méthylène étant préféré. Ou bien de façon alternative à faire réagir l'acide précurseur (IV) avec une amine (XI) dans un solvant inerte comme le chlorure de méthylène et en présence d'une carbodiimide comme la dicyclohexylcarbodiimide (DCCI) ou la [(diméthylamino-3)-propyl]-1-éthyl-3-carbodiimide qui sont les agents de condensation préférés.

L'amide intermédiaire (III) ainsi obtenu est mis en solution dans un solvant inerte aux réactifs utilisés et est alors réduit par un agent réducteur approprié pour donner l'amino alcool (II) obtenu sous forme d'un mélange d'isomères qui ne sont pas séparés. Les réducteurs utilisés pour la mise en oeuvre de cette étape intermédiaire sont choisis parmi la classe des hydrures métalliques ou organométalliques, plus précisément les hydrures dérivés du bore (BH₃), les hydrures dérivés de l'aluminium parmi lesquels on peut citer à titre d'exemples des hydrures d'aluminium simples comme AlH₃ ou le Dibal [(CH₃)₂CHCH₂]₂Al, des hydrures mixtes d'aluminium et de métaux alcalins comme le sodium ou le lithium, l'hydrure de lithium aluminium (LiAlH₄ ou LAH) étant préféré. Tel que montré au schéma 3 les amides (III.a) sont obtenus par réaction de condensation des intermédiaires (III) avec l'éthylène glycol, en présence d'un catalyseur acide comme l'acide p. toluène sulfonique. Egalement les alcools (II) dans lesquels R₁ est l'hydrogène sont avantageusement N-méthylés par alkylation réductrice avec le formaldéhyde et l'acide formique pour préparer les composés N-méthylés correspondants. Les acides (IV) sont connus et, à défaut d'être commercialisés sont fabriqués selon des procédés décrits comme entre autres aux publications J. Am. Chem. Soc. 1954, 76, p.4588; J. Agric. Food Chem. 1984, 32, p.1125-1129; Chem. Pharm. Bull. 1987, 35, p.1790-1795 ou encore par la méthode proposée par C.C.Chan and P.S.Farmer, Pharmazie (1986), 41(12),835-836.

Les amines (XI) sont accessibles dans le commerce, notamment celles pour lesquelles R₁ est l'hydrogène. Lorsque R₁ est alkyl de C₁ à C₄ les amines secondaires (XI) sont connues et peuvent être préparées selon des méthodes décrites dans l'état de la technique ou encore d'après la méthode proposée par F.F.Blicke, E.Monroe, J.Amer.Chem.Soc., 1939(61), p 91-95.

La présente invention est illustrée de façon non limitative par les exemples qui suivent.

L'état de pureté, les caractéristiques physico chimiques et l'identité structurelle des produits sont déterminés et rapportés comme suit :
- les produits sont purifiés par des techniques appropriées notamment par la chromatographie sur colonne pour laquelle on utilise favorablement la technique dite de "Chromatoflash" sur colonne de silice (marque "Merck", produit Kieselgel H 60, granulométrie 230 à 400 mesh). L'état de pureté des produits obtenus est déterminé par la méthode de chromatographie sur couche mince (CCM) de silice (plaques prêtes à l'emploi "Merck"); les Rf observés ainsi que les références des solvants d'élution utilisés sont indiqués dans les exemples.
- les caractéristiques physico chimiques des produits, sont représentées :
   a) par le point de fusion, déterminé par la méthode du tube capillaire et dont la valeur indiquée n'est pas corrigée
   b) par la spectrographie infra rouge (IR) des composés en pastilles de KBr; les absorptions les plus intenses sont rapportées par la valeur de leur nombre d'onde en cm⁻¹.
- l'identité structurelle des produits est déterminée en accord avec :
   a) la résonance magnétique nucléaire du proton (RMN) étudiée à 90 MHz, les produits étant solubilisés dans le deutérochloroforme en présence d'une trace d'hydroxyde de sodium lorsque le composé est étudié sous une forme salifiée, le plus couramment sous forme de chlorhydrate. L'aspect des signaux, leur déplacement chimique exprimé en p.p.m. par rapport au tétraméthylsilane utilisé comme référence interne sont indiqués. Les protons dits échangeables après addition d'oxyde de deutérium sont également signalés.
   b) l'analyse centésimale élémentaire dont les résultats, conformes aux normes admises ne sont pas reportés. Ces analyses sont signalées être effectuées par la représentation de l'élément dosé.

### EXEMPLE 1 : Chlorhydrate de 2-[2-[(N-cyclopropylméthyl-N-méthyl)amino]éthyl]-1-oxo-1,2-dihydroindène.

(Formule I.a; X=Y=H; R₁=CH₃; A=liaison de valence; m=1; n=2)

### Stade 1: N-cyclopropylméthyl-N-méthyl-2-[2-(1-oxo-1,2-dihydroindényl)]-acétamide.

(III; X=Y=H; R₁=CH₃; A=liaison de valence; m=1; n=2)
a) Dans un réacteur de 250 ml équipé en position de reflux et maintenu à l'abri de l'humidité par une garde à chlorure de calcium et sous atmosphère d'azote, on introduit 70 ml de chlorure de méthylène anhydre dans lequel on ajoute 6,8 g (35,7 mmole) d'acide 1-indanone-2-acétique.
   A la suspension obtenue, on ajoute sous agitation et goutte à goutte en 15 minutes une solution de 9,9 g (6,1 ml-83,0 mmole) de chlorure de thionyle dans 70 ml de chlorure de méthylène anhydre.
   Après cette introduction la suspension est chauffée au reflux durant une heure. La solution orangée obtenue est évaporée sous vide et sur bain marie. On obtient un résidu huileux orangé constitué par le chlorure de l'acide brut qui est engagé tel quel dans l'étape suivante.
b) Dans un réacteur de 500 ml équipé en position de reflux avec une garde de chlorure de calcium et sous atmosphère d'azote, on introduit 10,0 g (120,0 mmole) de N-cyclopropylméthylméthylamine en solution dans 70 ml de THF (tétrahydrofurane) anhydre puis on ajoute 70 ml de chlorure de méthylène anhydre.

Sous agitation et à une température comprise entre 25 et 30°C on ajoute goutte à goutte en 15 minutes le chlorure d'acide préparé au stade 1a) précédent, en solution dans 110 ml de chlorure de méthylène anhydre.

La solution orangée obtenue est chauffée sur bain d'huile au reflux que l'on maintient deux heures.

Après refroidissement à 20-25°C la solution est extraite successivement par :
- 50 ml d'une solution saturée en NaHCO₃
- 50 ml d'une solution HCl N
- deux fois 50 ml d'eau.

La phase organique est déshydratée sur Na₂SO₄ puis les solvants sont éliminés par distillation sous vide et sur bain marie à 50 °C.

On obtient un résidu huileux de 8,0 g que l'on purifie par "Chromatoflash".

L'élution par l'acétate d'éthyle permet d'obtenir 5,6 g de N-cyclopropylméthyl-N-méthyl-2-[2-(1-oxo-1,2-dihydroindényl)]-acétamide purifié. Rdt = 60,9%
- CCM : Rf = 0,70 (acétate d'éthyle)

### Stade 2 : 2-[2-[(N-cyclopropylméthyl-N-méthyl)amino]éthyl]-1-hydroxy-1,2-dihydroindène.;

(isomères II; X=Y=H; R₁=CH₃; A=liaison de valence; m=1; n=2)

Dans un réacteur de 250 ml équipé en position de reflux avec une garde de chlorure de calcium et sous atmosphère d'azote, on introduit 55 ml d'éther diéthylique anhydre et 3,1 g (81,6 mmole) d'hydrure de lithium aluminium (LAH).

A la suspension obtenue on ajoute sous agitation, goutte à goutte et en 15 minutes une solution de 5,6 g (21,8 mmole) de l'amide obtenue au stade 1b) précédent en solution dans 55 ml d'éther.

L'introduction est exothermique et se termine au reflux de l'éther. On chauffe ensuite au bain d'huile pour maintenir ce reflux pendant deux heures.

A la suspension grise qui est refroidie à une température inférieure à 10°C on ajoute successivement goutte à goutte et avec précautions :
- 3,1 ml d'eau,
- 3,1 ml de solution NaOH à 15% (p/v)
- 7,0 ml d'eau.

La suspension blanche obtenue est maintenue à température ambiante sous agitation une heure et demie, l'insoluble est alors filtré sur büchner et lavé à l'éther.

Les phases éthérées sont réunies et évaporées sous vide sur bain marie à 50 °C.

On obtient 4,5 g de produit brut résiduel qui se présente sous la forme d'une huile colorée que l'on purifie par "Chromatoflash".

L'élution par un mélange chlorure méthylène-méthanol à 10 % d'ammoniaque (95/5-v/v) permet de purifier le produit sous la forme d'une huile jaune, constitué par un mélange d'isomères.
Poids = 4,0 g Rdt = 74,9%
- CCM : Rf = 0,30 et 0,50 (majoritaire) (chlorure de méthylène-methanol, NH₄OH 10%-95/5-v/v).

### Stade 3 : 2-[2-[(N-cyclopropylméthyl-N-méthyl)amino]éthyl]-1-oxo-1,2-dihydroindène.

(I.a; X=Y=H; R₁=CH₃; A=liaison de valence; m=1; n=2)

Dans un ballon on introduit 4,0 g (16,3 mmole) du mélange d'isomères d'amino alcools préparé au stade 2 précédent et 190 ml de chlorure de méthylène anhydre. Sous agitation, on ajoute 9,25 g (43,0 mmole) de chlorochromate de pyridinium.

Le mélange noirâtre obtenu est maintenu sous agitation 16 heures à 20-25°C. Après repos la phase chlorure de méthylène surnageante est décantée, la gomme résiduelle est traitée à nouveau par du chlorure de méthylène que l'on décante également. Les phases organiques réunies sont filtrées sur terre d'infusoire puis extraites par deux fois 100 ml de solution NaOH N.

La phase alcaline est écartée, on ajoute 250 ml d'éther à la phase organique. Le précipité qui se forme est filtré sur terre d'infusoire et le filtrat concentré sous vide sur bain marie à 50°C. Le résidu est repris par 150 ml d'hexane, l'insoluble filtré, le filtrat est extrait à deux reprises par deux fois 25 ml de solution HCl 10% (v/v)

La phase organique est écartée, et la phase acide alcalinisée à une température voisine de 10°C jusqu'à pH 12 par une solution NaOH 10 N.

Le mélange alcalin est extrait par trois fois 50 ml d'éther.

Les phases éthérées réunies sont lavées à l'eau puis déshydratées sur Na₂SO₄. L'éther est éliminé par distillation sous vide sur bain marie à 50 °C. On obtient le produit sous forme d'huile incolore dans un état de pureté satisfaisant après examen en CCM.
Poids = 3,5 g Rdt = 88,2%
- CCM : Rf = 0,25-0,40 (chlorure de méthylène-méthanol, NH₄OH 10%-95/5-v/v).

### Préparation du chlorhydrate

L'amino-cétone obtenue ci-dessus est solubilisée dans 35 ml de chlorure de méthylène anhydre. On ajoute à 10 °C environ 5 ml d'éther chlorhydrique environ 5 N puis on chasse les solvants par distillation sous vide et sur bain marie à 50 °C. Le résidu est dissout dans 20 ml d'isopropanol. On ajoute 50 ml d'éther pour précipitation du chlorhydrate. L'insoluble est filtré, lavé à l'éther et séché sous vide. Poids = 3,5 g

Le produit est repris pour purification par 15 ml d'isopropanol et 25 ml d'éther. L'insoluble est filtré, lavé à l'éther puis séché.
Poids = 3,0 g Rdt = 74,6% F = 160-162°
- CCM : Rf = 0,30 -0,40 (chlorure de méthylène-méthanol,NH₄OH 10%-95/5-v/v).
- Analyse (C₁₆H₂₂ClNO) C,H,Cl,N,O
- IR (KBr) : 2900, 2500, 1710, 1610, 1460, 1204, 1020, 750 cm⁻¹
- RMN : 0-0,2(m,2H) ; 0,3-0,6(m, 2H) ; 0,6-1(m,1H) ; 1,3-3,5 (m,9H) ; 2,3(s,3H) ; 7,2-7,8 (m,4H)

### EXEMPLE 2 : Chlorhydrate de 2-[2-[(N-cyclopropylméthyl-N-méthyl)amino]éthyl]-1-oxo-1,2,3,4-tétrahydronaphtalène.

(Formule I.a; X=Y=H; R₁=CH₃; A=CH₂; m=1; n=2)

### Stade 1 : N-cyclopropylméthyl-N-méthyl-2-[2-(1-oxo-1,2,3,4-tétrahydronaphtyl)]-acétamide.

(III; X=Y=H; R₁=CH₃; A=CH₂; m=1; n=2)
a) Dans un réacteur d'un litre équipé en position de reflux sous atmosphère d'azote et protégé de l'humidité par une garde à chlorure de calcium, on introduit 320 ml de chlorure de méthylène anhydre dans lequel on ajoute 30,6g (150 mmole) d'acide 1-oxo-1,2,3,4-tétrahydro-2-naphtalène acétique.
   La solution orangée obtenue est mise sous agitation puis on ajoute goutte à goutte en 10 minutes une solution de 41,5 g (25,3 ml-35 mmole) de chlorure de thionyle en solution dans 320 ml de chlorure de méthylène anhydre.
   Après cette introduction la solution est chauffée sur bain d'huile et portée au reflux que l'on maintient durant une heure. La solution orangée obtenue est d'abord refroidie puis évaporée sous vide au bain marie à 50°C. On obtient un résidu coloré de 30,0 g constitué par le chlorure de l'acide brut que l'on engage tel que dans la phase suivante.
b) Dans un réacteur d'un litre équipé en position de reflux sous atmosphère d'azote, on introduit 25,5 g (300 mmole) de N-cyclopropylméthyl-méthylamine en solution dans du THF anhydre puis on ajoute 450 ml de chlorure de méthylène anhydre.

Sous agitation et à température ambiante on ajoute goutte à goutte en 15 minutes le chlorure d'acide précédemment préparé au stade 1a), en solution dans 320 ml de chlorure de méthylène anhydre.

La solution marron obtenue est chauffée au reflux sur bain d'huile et maintenue au reflux pendant trois heures. Après refroidissement à température ambiante le mélange est lavé par 250 ml d'une solution de NaHCO₃.

La phase organique surnageante est extraite par 250 ml d'une solution de HCl 10% puis lavée par 250 ml d'eau.

La phase organique est ensuite déshydratée sur Na₂SO₄ et les solvants sont éliminés par distillation sous vide et sur bain marie à 50°C.

On obtient un résidu (huileux) de 39,0 g que l'on purifie par "Chromatoflash".

L'élution par l'acétate d'éthyle permet d'obtenir 26,8 g de N-cyclopropylméthyl-N-méthyl-2-[2-(1-oxo-1,2,3,4-tétrahydronaphtyl)]-acétamide purifié. Rdt =65,9%
- CCM : Rf 0,80 (acétate d'éthyle)

### Stade 2 : 2-[2-[(N-cyclopropylméthyl-N-méthyl)amino]éthyl]-1-hydroxy-1,2,3,4-tétrahydronaphtalène.

(isomères II; X=Y=H; R₁=CH₃; A=CH₂; m=1; n=2)

Dans un réacteur d'un litre équipé en position de reflux , protégé de l'humidité par une garde à chlorure de calcium et sous atmosphère d'azote, on introduit sous agitation 250 ml d'éther diéthylique anhydre et 13,9 g (367,0 mmole) d'hydrure de lithium aluminium (LAH).

A la suspension obtenue on ajoute sous agitation, goutte à goutte et en 30 minutes une solution de 26,8 g (98,8 mmole) de l'amide obtenue au stade 1b) précédent, en solution dans 250 ml d'éther.

L'introduction est exothermique et se termine au reflux de l'éther. On chauffe ensuite la solution dans un bain d'huile pour maintenir ce reflux pendant 45 minutes.

A la suspension obtenue qui est refroidie jusqu'à une température inférieure à 0°C on ajoute successivement goutte à goutte et avec précautions :
- 13,9 ml d'eau déminéralisée
- 13,9 ml de solution NaOH à 15% (p/v)
- 31 ml d'eau déminéralisée.

La suspension obtenue est maintenue 30 minutes sous agitation à 0°C, puis l'insoluble est filtré sous vide sur büchner garni de terre infusoire.

Le filtrat est évaporé sous vide sur bain marie à 50 °C.

Le produit brut résiduel obtenu pèse 23,3 g et est purifié par "Chromatoflash".

L'élution par un mélange chlorure de méthylène-méthanol à 10 % d'ammoniaque (95/5-v/v) permet de purifier le produit sous forme d'une huile jaune, constitué par un mélange d'isomères.
Poids = 9,8 g Rdt = 38,2%
- CCM : Rf= 0,50-0,70 (chlorure de méthylène-méthanol, NH₄OH 10%-95/5-v/v).

### Stade 3 : 2-[2-[(N-cyclopropylméthyl-N-méthyl)amino]éthyl]-1-oxo-1,2,3,4-tétrahydronaphtalène.

(I.a; X=Y=H; R₁=CH₃; A=CH₂; m=1; n=2)

Dans un réacteur d'un litre équipé en position de reflux sous atmosphère d'azote et protégé de l'humidité par une garde à chlorure de calcium, on introduit 9,7 g (37,4 mmole) du mélange d'isomères d'amino alcools préparé au stade 2 précédent en solution dans 430 ml de chlorure de méthylène anhydre. Sous agitation, on ajoute 21,2 g (98,0 mmole) de chlorochromate de pyridinium.

Le mélange noirâtre obtenu est maintenu 20 heures sous agitation à température ambiante. La phase surnageante est décantée et filtrée sur terre d'infusoire. La gomme résiduelle est extraite par deux fois 150 ml de chlorure de méthylène, que l'on décante et filtre sur terre d'infusoire. Les phases organiques sont extraites successivement par trois fois 150 ml de solution NaOH N puis par 150 ml d'eau. Les phases aqueuses sont écartées et on ajoute 500 ml d'éther à la phase organique obtenue. Le précipité qui se forme est filtré sur terre d'infusoire et le filtrat concentré sous vide sur bain marie à 50°C. Le résidu obtenu est repris dans 150 ml d'hexane.

L'insoluble floconneux est filtré et le filtrat contenant la phase hexane est extrait par deux fois 75 ml de solution HCl 10%.

La phase organique est écartée, la phase acide alcalinisée à froid jusqu'à pH 12 par une solution de NaOH.

Le mélange alcalin est extrait par trois fois 100 ml d'éther.

Les phases éthérées réunies sont lavées à l'eau puis déshydratées sur Na₂SO₄. L'éther est éliminé par distillation sous vide sur bain marie à 50 °C. On obtient 7,7 g de 2-[2-[(N-cyclopropylméthyl-N-méthyl)amino]éthyl]-1-oxo-1,2,3,4-tétrahydronaphtalène sous la forme d'une huile violacée.
Rdt = 80%
- CCM : Rf= 0,60 (chlorure de méthylène-méthanol, NH₄OH 10%-80/20-v/v)

### Préparation du chlorhydrate

L'amino-cétone obtenue ci-dessus est solubilisée dans 80 ml d'éther. Après refroidissement de la solution, on ajoute 12 ml d'éther chlorhydrique environ 5 N et on chasse les solvants par distillation sous vide sur bain marie à 50°C. Le résidu de couleur violette obtenu est dissous dans 25 ml d'isopropanol.

On ajoute 50 ml d'éther au chlorhydrate qui précipite lentement. Après 48 heures au froid et au repos, l'insoluble est filtré, lavé à l'éther et séché sous vide. Poids = 7,0 g Le précipité est repris pour purification par 25 ml d'isopropanol et 50 ml d'éther. La solution est laissée deux heures à froid sous agitation, l'insoluble est filtré puis lavé à l'éther et séché sous vide à 60°C.
Poids = 6,0 g Rdt = 68,3% F = 123-125°
- CCM : Rf = 0,50-0,65 (chlorure de méthylène-méthanol,NH₄OH 10%-80/20-v/v)
- Analyse (C₁₇H₂₄ClNO) C,H,Cl,N,O
- IR (KBr) : 2920, 2005, 1680, 1600, 1422, 1220, 1000, 740 cm⁻¹
- RMN : 0-0,2(m,2H) ; 0,3-0,6(m,2H) ; 0,6-1(m,1H) ; 2,3(s,3H) ; 1,3-3,1(m,11H) ; 7,1-8,1(m,4 H)

### EXEMPLE 3 : Chlorhydrate de 2-[2-[(N-cyclobutylméthyl-N-méthyl)amino]éthyl]-1-oxo-1,2,3,4-tétrahydronaphtalène.

(Formule I.a; X=Y=H; A=CH₂; R₁=CH₃; m=1; n=3)

Selon le mode opératoire décrit à l'exemple 2 précédent, à partir d'acide 1-oxo-1,2,3,4-tétrahydro-2-naphtalène acétique et de N-cyclobutylméthyl-méthylamine on obtient successivement aux stades 1 à 3 les composés suivants.

### Stade 1: N-cyclobutylméthyl-N-méthyl-2-[2-(1-oxo-1,2,3,4-tétrahydronaphtyl)]-acétamide. (III; X=Y=H; A=CH₂; R₁=CH₃; m=1; n=3) Rdt = 65,6%

- CCM : Rf = 0,80 (acétate d'éthyle)

### Stade 2 : 2-[2-[(N-cyclobutylméthyl-N-méthyl)amino]éthyl]-1-hydroxy-1,2,3,4-tétrahydronaphtalène.

(isomères II; X=Y=H; A=CH₂; R₁=CH₃; m=1; n=3)Rdt = 91,5%
- CCM :Rf = 0,50 ; majoritaire 0,70 (chlorure de méthylène-méthanol,NH₄OH 10%-95/5-v/v)

### Stade 3 : 2-[2-[(N-cyclobutylméthyl-N-méthyl)amino]éthyl]-1-oxo-1,2,3,4-tétrahydronaphtalène. (I.a; X=Y=H; A=CH₂; R₁=CH₃; m=1; n=3) Rdt = 83,9% (huile violette)

- CCM : Rf= 0,60 (chlorure de méthylène -méthanol, NH₄OH 10%-95/5-v/v)

### Chlorhydrate

Rdt = 52,9% F = 120-121°C
- CCM : Rf = 0,40-0,60 (chlorure de méthylène -méthanol, NH₄OH 10%-95/5-v/v)
- Analyse (C₁₈H₂₆ClNO) C, H, Cl, N, O
- IR (KBr) : 2900, 2500, 1680, 1600, 1450, 1280, 1210, 1105, 740 cm⁻¹
- RMN : 1,25-2,8(m,15H) ; 2,2(s,3H) ; 2,85-3,20(m,2H); 7,1-8,1 (m,4H)

### EXEMPLE 4 : Chlorhydrate de 2-[2-[(N-cyclopropyléthyl-N-méthyl)amino]éthyl]-1-oxo-1,2,3,4-tétrahydronaphtalène.

(Formule I.a; X=Y=H; A=CH₂; R₁=CH₃; m=2; n=2)

Selon le mode opératoire décrit à l'exemple 2 précédent, à partir d'acide 1-oxo-1,2,3,4-tétrahydro-2-naphtalène acétique et de N-cyclopropyléthyl-méthyl amine on obtient successivement aux stades 1 à 3 les composés suivants.

### Stade 1 : N-cyclopropyléthyl-N-méthyl-2-[2-(1-oxo-1,2,3,4-tétrahydronaphtyl)]-acétamide. (III; X=Y=H; A=CH₂; R₁=CH₃; m=2; n=2) Rdt = 64,8%

- CCM : Rf= 0,80 (acétate d'éthyle)

### Stade 2 : 2-(2-[(N-cyclopropyléthyl-N-méthyl)amino]éthyl]-1-hydroxy-1,2,3,4-tétrahydronaphtalène. (isomères II; X=Y=H; A=CH₂; R₁=CH₃; m=2; n=2) Rdt = 93%

- CCM : Rf = 0,20 (chlorure de méthylène -méthanol, NH₄OH 10%-95/5-v/v)

### Stade 3 : 2-[2-[(N-cyclopropyléthyl-N-méthyl)amino]éthyl]-1-oxo-1,2,3,4-tétrahydronaphtalène. (I.a; X=Y=H; A=CH₂; R₁=CH ₃; m=2; n=2) Rdt = 73,4% (huile verdâtre)

- CCM : Rf= 0,60-0,70 (chlorure de méthylène-méthanol, NH₄OH 10%-95/5-v/v)

### Chlorhydrate

Rdt = 44,1% F = 103-106°C
- CCM : Rf = 0,50 (chlorure de méthylène -méthanol, NH₄OH 10%-95/5-v/v)
- Analyse (C₁₈H₂₆ClNO) C, H, Cl, N, O
- IR (KBr) : 2900, 2800, 1680, 1600, 1450, 1360, 1210, 1005, 740 cm⁻¹
- RMN : 0-0,1(m,2 H) ; 0,4-0,5(m,2H) ; 0,5-0,8(m,1H) ; 1,2-2,7(m,11H) ; 2,8-3,1(m,2H) ; 7,1-8,1(m,4H)

### EXEMPLE 5 : Chlorhydrate de 2-[2-[(N-cyclohexylméthyl-N-méthyl)amino]éthyl]-1-oxo-1,2,3,4-tétrahydronaphtalène.

(Formule I.a; X=Y=H; R₁=CH₃; A=CH₂; m=1; n=5)

Le composé est préparé tel que décrit à l'exemple 2 à partir d'acide 1-oxo-1,2,3,4-tétrahydro-2-naphtalène acétique et de N-cyclohexylméthyl-méthylamine.

### Stade 1 : N-cyclohexylméthyl-N-méthyl-2-[2-(1-oxo-1,2,3,4-tétrahydronaphtyl)]-acétamide. (III.a ; X=Y=H; R₁=CH₃; A=CH₂; m=1; n=5) Rdt = 53%

- CCM : Rf = 0,80 (huile jaune après purification chromatographique par l'acétate éthyle)

### Stade 2 : 2-[2-[(N-cyclohexylméthyl-N-méthyl)amino]éthyl]-1-hydroxy-1,2,3,4-tétrahydronaphtalène. (isomères II; X=Y=H; R₁=CH₃; A=CH₂; m=1; n=5) Rdt = 83,5%

- CCM : Rf = 0,70 et 0,80 (chlorure de méthylène - méthanol ammoniacal 10% - 95/5 -v/v)

### Stade 3: 2-[2-[(N-cyclohexylméthyl-N-méthyl)amino]éthyl]-1-oxo-1,2,3,4-tétrahydronaphtalène. (I.a; X=Y=H; R₁=CH₃; A=CH_{2;} m=1; n=5) Rdt = 75%

- CCM : Rf = 0,60-0,70 (chlorure de méthylène - méthanol ammoniacal 10% - 95/5 -v/v)

### chlorhydrate

Rdt = 80% F = 165°C
CCM : Rf = 0,25-0,45 (chlorure de méthylène - méthanol ammoniacal 10% - 95/5 -v/v)
- Analyse (C₂₀H₃₀ClNO) C, H, Cl, N, O
- IR (KBr) : 2900, 2400, 1680, 1600, 1440, 1220, 900, 740 cm⁻¹
- RMN : 0,5-2,8(m,20H) ; 2,15(s,3H) ; 2,8-3,1(m,2H) ; 7,1-8,1(m,4H)

### EXEMPLE 6 : Chlorhydrate de 2-[2-[(N-cyclohexylméthyl-N-méthyl)amino]éthyl]-6-méthoxy-1-oxo-1,2,3,4-tétrahydronaphtalène.

(Formule I.a; X=6-OCH₃; Y=H; R₁=CH₃; A=CH₂; m=1; n=5)

### Stade 1: N-cyclohexylméthyl-N-méthyl-2-[2-(6-méthoxy-1-oxo-1,2,3,4-tétrahydronaphtyl)]-acétamide. (III; X=OCH₃; Y=H; R₁=CH₃; A=CH₂; m=1; n=5)

Dans un réacteur protégé de l'humidité dans 60ml de chlorure de méthylène deshydraté sur tamis, on introduit 1,00g (4,3mmole) d'acide 1-oxo-6-méthoxy-1,2,3,4-tétrahydro-2-naphtalène acétique, 0,68g (5,4 mmole) de N-cyclohexylméthyl-méthylamine et 1,33g (6,45 mmole) de dicyclohexyl-carbodiimide (DCCI).

Après dissolution le mélange est agité durant 1 h 30 à 20-25°C puis traité. On obtient 1,7g de produit brut qui est repris par 20 ml d'éther anhydre. L'insoluble est filtré et éliminé. La phase éthérée est évaporée et le résidu purifié par "Cromatoflash". L'élution par un mélange acétate d'éthylehexane 70-30 permet d'obtenir le produit purifié - Poids 0,70g. Rdt = 47,5%
- CCM : Rf = 0,60-0,70 (Acétate d'éthyle-hexane-70-30-v/v)

### Stade 2: 2-[2-[(N-cyclohexylméthyl-N-méthyl)amino]éthyl]-6-méthoxy-1-hydroxy-1,2,3,4-tétrahydronaphtalène. (isomères II; X=6-OCH₃;Y=H; R₁=CH₃; A=CH₂; m=1; n=5)

Préparé à partir du produit du stade précédent et selon le mode opératoire décrit à l'exemple 2 - stade 2. Rdt = 92,1%
- CCM : Rf = 0,15-0,40 (chlorure de méthylène-méthanol ammoniacal 10%-97-3-v/v)

### Stade 3 : 2-[2-[(N-cyclohexylméthyl-N-méthyl)amino]éthyl]-6-méthoxy-1-oxo-1,2,3,4-tétrahydronaphtalène. (I.a; X=6-OCH₃; Y=H; R₁=CH₃; A=CH₂; m=1; n=5)

Préparé selon le mode opératoire décrit à l'exemple 2-stade 3. Rdt = 73,1%
- CCM : Rf = 0,20-0,30 (chlorure de méthylène - méthanol ammoniacal 10% - 97/3 v/v)

### chlorhydrate

Rdt = 73% F **=** 172°C
CCM : Rf = 0,25 (chlorure de méthylène-méthanol ammoniacal 10%-97-3-v/v)
- Analyse (C₂₁H₃₂ClNO₂) C, H, Cl, N, O
- IR (KBr) : 2900, 2550, 1675, 1600, 1440, 1250, 1100, 840, 760 cm⁻¹
- RMN : 0,6-2,7(m,2OH) ; 2,2(s,3H) ; 2,8-3,1(m,2H) ; 3,8(s,3H); 6,6-6,9(m,2H) ; 8,0(d,1H)

### EXEMPLE 7 : 2-[2-(N-cyclopropylamino)éthyl]-1-oxo-1,2,3,4-tétrahydronaphtalène.

(Formule I.a; X=Y=H; R₁=H; A=CH₂; m=0; n=2)

### Stade 1 : N-cyclopropyl-2-[2-(1-oxo-1,2,3,4-tétrahydronaphtyl)]-acétamide. (III; X=Y=H; R₁=H; A=CH₂; m=0; n=2)

Le composé est préparé tel que décrit à l'exemple 6 - stade 1 précédent à partir d'acide 1-oxo-1,2,3,4-tétrahydro-2-naphtalène acétique et de cyclopropylamine. Rdt = 86%
- CCM : Rf = 0,10-0,20 (Acétate d'éthyle-hexane-70-30-v/v))

### Stade 2 : 2-[2-(N-cyclopropylamino)éthyl]-1-hydroxy-1,2,3,4-tétrahydronaphtalène. (isomères II; X=Y=H; R₁=H; A=CH₂; m=0; n=2)

Préparé à partir du produit du stade précédent et selon le mode opératoire décrit à l'exemple 2 - stade 2. Rdt = 80%
- CCM : Rf = 0,20-0,30 (Chlorure de méthylène-méthanol ammoniacal 10% - 95/5 v/v)

### Stade3: 2-[2-(N-cyclopropylamino)éthyl]-1-oxo-1,2,3,4-tétrahydronaphtalène. (I.a; X=Y=H; R₁=H; A=CH₂; m=0; n=2)

Préparé selon le mode opératoire décrit à l'exemple 2-stade 3 Rdt = 14,0% (instable)
- CCM : Rf = 0,15-0,25 (Chlorure de méthylène-méthanol ammoniacal à 10 %-90/10-v/v)
- Analyse (C₁₅H₁₉NO) C, H, N, O
- IR (KBr) : 2900,2850,1680,1600,1450,1280,1220,1030,740cm⁻¹
- RMN : 0,0-0,1(m,2H);0,2-0,6(m,3H);1,65-2,35(m,5H);2,5-3,25 (m,5H dont 1 ech.);7,1-7,6(3,3H);7,9-8,1(m,1H)

### EXEMPLE 8 : Chlorhydrate de 2-[2-[(N-cyclopropyl-N-méthyl)amino]éthyl]-1-oxo-1,2,3,4-tétrahydronaphtalène.

(Formule I.a; X=Y=H; R₁=CH₃; A=CH₂; m=0; n=2)

### Stade 1: 2-[2-[(N-cyclopropyl-N-méthyl)amino]éthyl]-1-hydroxy-1,2,3,4-tétrahydronaphtalène. (Isomères II; X=Y=H; R₁=CH₃; A=CH₂; m=0; n=2)

Dans un ballon on introduit 15,0 g (65,2 mmole) de l'amine obtenue à l'exemple 7 - stade 2 précédent. On ajoute ensuite en refroidissant 9,4 g (196 mmole) d'acide formique pur puis 13,3g de solution aqueuse de formaldéhyde à 37% p/v. Le mélange est maintenu sous agitation durant 24 h à 80°C puis il est refroidi et on y ajoute 19,6ml de solution HCl N. Après extractions par 3 fois 30 ml d'éther la phase aqueuse est alcalinisée à froid par une solution NaOH 10N puis extraite à l'éther. Après évaporation du solvant on obtient le produit brut sous forme d'une huile (Rdt 92%). Le produit est purifié par chromatographie sur colonne de silice et par élution sélective avec l'acétate d'éthyle. Rdt = 72%
- CCM : Rf = 0,35-0,50 (Acétate d'éthyle)

### Stade 2: 2-[2-[(N-cyclopropyl-N-méthyl)amino] éthyl]-1-oxo-1,2,3,4-tétrahydronaphtalène. (I.a; X=Y=H; R₁=CH₃; A=CH₂; m=0; n=2)

Préparé à partir du produit du stade précédent et selon le mode opératoire décrit à l'exemple 2 - stade 3. Rdt = 67%
- CCM : Rf = 0,70-0,90 (Chlorure de méthylène-méthanol ammoniacal à 10% 95-5 v/v)

### Chlorhydrate

Rdt = 77% F = 167-169°C
- CCM : Rf = 0,65-0,75 (Chlorure de méthylène-méthanol ammoniacal à 10% 95-5 v/v)
- Analyse (C₁₆H₂₁ClNO) C, H, Cl,N, O
- IR (KBr) : 2900, 2600, 2450, 1675, 1600, 1410, 1220, 1030, 740 cm⁻¹
- RMN : 0,2-0,55 (m,4H);1,35-2,8(m,8H);2,35(S,3H);2,85-3,1(m,2H);7,1-7,6(m,3H);7,9-8,15(m,1H)

### EXEMPLE 9 : Chlorhydrate de 2-[2-[(N-cyclopropylméthyl-N-propyl)amino]éthyl]-1-oxo-1,2,3,4-tétrahydronaphtalène.

(Formule I.a; X=Y=H; R₁=nC₃H₇; A=CH₂; m=1; n=2)

### Stade 1 : N-cyclopropylméthyl-N-propyl-2-[2-(1-oxo-1,2,3,4-tétrahydronaphtyl)]-acétamide. (III; X=Y=H; R₁=C₃H₇; A=CH_{2;} m=1; n=2)

Le composé est préparé tel que décrit à l'exemple 6 - stade 1 à partir d'acide 1-oxo-1,2,3,4-tétrahydro-2-naphtalène acétique et de N-cyclopropylméthyl-propylamine.

Le produit brut est purifié par chromatographie sur colonne en éluant par un mélange acétate d'éthyle - hexane. Rdt = 65%
- CCM : Rf = 0,60-0,65 (Acétate d'éthyle-hexane 70-30 v/v)

### Stade 2 : 2-[2-[(N-cyclopropylméthyl-N-propyl)amino]éthyl]-1-hydroxy-1,2,3,4-tétrahydronaphtalène. (isomères II; X=Y=H; R₁=nC₃H₇; A=CH₂; m=1; n=2)

Préparé à partir du produit du stade précédent et selon le mode opératoire décrit à l'exemple 2 - stade 2. Rdt = 89%
- CCM : Rf = 0,10-0,50 (Chlorure de méthylène-méthanol ammoniacal 10% 95-5 v/v)

### Stade 3 : 2-[2-[(N-cyclopropylméthyl-N-propyl)amino]éthyl]-1-oxo-1,2,3,4-tétrahydronaphtalène. (I.a; X=Y=H; R₁=nC₃H₇; A=CH₂; m=1; n=2)

Préparé selon le mode opératoire décrit à l'exemple 2-stade 3.

Le produit est purifié par chromatographie sur colonne en éluant par un mélange chlorure de méthylène - méthanol ammoniacal à 10%. Rdt = 55,0%
- CCM : Rf = 0,30-0,60 (Chlorure de méthylène-méthanol ammoniacal 10% 95-5 v/v)

### chlorhydrate

Rdt = 60% F = 98-100°C
- CCM : Rf = 0,40 (Chlorure de méthylène-méthanol ammoniacal 10% 95-5 v/v)
- Analyse (C₁₉H₂₈ClNO) C, H, Cl, N, O
- IR (KBr) : 2900,2450,1670,1600,1470,1450,1270,1220,730cm⁻¹
- RMN : 0,0-0,2(m,2H);0,3-0,6(m,2H);0,6-1,0(m,4H);
   1,1-2,8(m,13H);2,8-3,15(m,2H);7,1-7,6(m,3H);7,9-8,2(m,1H)

### EXEMPLE 10 : Chlorhydrate de 2-[2-[(N-cyclopropylméthyl-N-méthyl)amino]éthyl]-1-benzosubérone.

(Formule I.a; X=Y=H; R₁=CH₃; A=CH₂-CH₂; m=1; n=2)

Le composé est préparé à partir d'acide 1-benzosubérone-2-acétique et de N-cyclopropylméthyl-méthylamine comme décrit aux stades 1, 2 et 3 de l'exemple 2.

### Stade 1: N-cyclopropylméthyl-N-méthyl-2-[(1-benzosubérone)-2-yl]-acétamide. (III; X=Y=H; R₁=CH₃; A=CH₂-CH₂; m=1; n=2) Rdt = 42,8%

- CCM : Rf = 0,80 (huile après purification chromatographique acétate d'éthyle)

### Stade 2 : 2-[2-[(N-cyclopropylméthyl-N-méthyl)amino]éthyl]-1-benzosubérol. (isomères II; X=Y=H; R₁=CH₃; A=CH₂-CH₂; m=1; n=2) Rdt = 80,1%

- CCM : Rf = 0,60 et 0,65 (chlorure méthylène - méthanol ammoniacal 95-5)

### Stade 3 : 2-[2-[(N-cyclopropylméthyl-N-méthyl)amino]éthyl]-1-benzosubérone. (I.a; X=Y=H; R₁=CH₃; A=CH₂-CH₂; m=1; n=2) Rdt = 75,6%

- CCM : Rf = 0,70-0,80 (chlorure méthylène - méthanol ammoniacal 95-5)

### chlorhydrate

Rdt = 55% F = 97-99°C
- CCM : Rf = 0,50-0,60 (chlorure méthylène - méthanol ammoniacal 95-5)
- Analyse (C₁₈H₂₆ClNO) C, H, Cl, N, O
- IR (KBr) : 2900, 2590, 2500, 1670, 1600, 1440, 1280, 1100, 960, 740 cm⁻¹
- RMN : 0-0,15(m,2H) ; 0,3-0,6(m,2H) ; 0,6-0,9(m,1H) ; 1,4-2,5(m,11H) ; 2,3(s,3H) ; 2,8-3,1(m,2H) ; 7,1-7,7(m,4H)

### EXEMPLE 11 : Chlorhydrate de 2-[2-[(N-cyclohexylméthyl-N-méthyl)amino]éthyl]-1-benzosubérone.

(Formule I.a; X=Y=H; R₁=CH₃; A=CH₂-CH₂; m=1; n=5)

Préparé comme décrit à l'exemple 10 précédent à partir de l'acide 1-benzosubérone-2-acétique et de la N-cyclohexylméthyl-méthylamine.

### Stade 1 : N-cyclohexylméthyl-N-méthyl-2-[(1-benzosubérone)-2-yl]-acétamide. (III; X=Y=H; R₁=CH₃; A=CH₂-CH₂; m=1; n=5) Rdt = 89,9%

- CCM : Rf = 0,70 (chlorure méthylène-méthanol ammoniacal 95-5)

### Stade 2 : 2-[2-[(N-cyclohexylméthyl-N-méthyl)amino]éthyl]-1-benzosubérol. (isomères II; X=Y=H; R₁=CH₃; A=CH₂-CH₂; m=1; n=5) Rdt = 95,2%

- CCM : Rf = (0,30-0,40) (chlorure de méthylène-méthanol 99-1 v/v)

### Stade 3 : 2-[2-[(N-cyclohexylméthyl-N-méthyl)amino]éthyl]-1-benzosubérone. (I.a; X=Y=H; R₁=CH₃; A=CH₂-CH₂; m=1; n=5) Rdt = 73,9%

- CCM : Rf = 0,40-O,50 (chlorure de méthylène-méthanol 99/1 v/v)

### chlorhydrate

Rdt = 92,8% F = 138-140°C
- CCM : Rf = 0,45 (chlorure de méthylène-méthanol 99/1 v/v)
- Analyse (C₂₁H₃₂ClNO) C, H, Cl, N, O
- IR (KBr) : 2900, 2600, 1675, 1600, 1440, 1280, 720 cm⁻¹
- RMN : 0,6-2,5(m,22H) ; 2,2(s,3H) ; 2,8-3,1(m,2H) ; 7,0-7,7 (m,4H)

### EXEMPLE 12 : 2-[2-[(N-cyclopropylméthyl-N-méthyl)amino]éthyl]-1-[spiro(cyclodioxyéthyl)]-1,2,3,4-tétrahydronaphtalène.

(Formule I.c; X=Y=H; R₁=CH₃; A=CH₂;m=1;n=2)

### Stade 1 : N-cyclopropylméthyl-N-méthyl-2-[2-[1-[spiro(cyclodioxyéthyl)] -1,2,3,4-tétrahydronaphtyl]-acétamide. (III.a; X=Y=H; R₁=CH₃;A=CH₂;m=1;n=2)

Dans un réacteur équipé avec un réfrigérant en position de reflux muni d'un système d'élimination d'eau de Dean Starck, on dissout dans 260 ml de toluène, 6,6g (24,3 mmole) de N-cyclopropylméthyl-N-méthyl-2-[2-(1-oxo-1,2,3,4-tétrahydronaphtyl)]-acétamide tel que préparé au stade 1 de l'exemple 2, 29,7 ml d'éthylène glycol et 0,22 g d'acide p. toluène sulfonique monohydrate.

Le mélange est porté au reflux avec élimination de l'eau formée. L'avancement est suivi par chromatographie en phase gazeuse. Après 40 h de reflux 27 ml d'eau ont été éliminés et l'avancement déterminé par CPG est de 80% de produit formé.

La solution est refroidie, extraite par 150ml d'une solution saturée en bicarbonate de sodium puis lavée par 2 fois 150ml d'eau. La phase toluènique est déshydratée sur Na₂SO₄ et le solvant évaporé.

Le résidu huileux verdâtre (7,0 g) est purifié par "Chromatoflash". L'élution par un mélange acétate d'éthylehexane permet de séparer 3,6g de produit purifié, Rdt = 47,0%
- CCM : Rf = 0,40-0,50 (acétate d'éthyle)

### Stade 2 : 2-[2-[(N-cyclopropylméthyl-N-méthyl)amino]éthyl]-1-[spiro(cyclodioxyéthyl)]-1,2,3,4-tétrahydronaphtalène.

(I.c; X=Y=H; R₁=CH₃;A=CH₂;m=1;n=2)

L'amide est réduit dans l'éther par l'hydrure de lithiumaluminium dans les conditions habituelles. Rdt = 90,6%
- CCM : Rf = 0,50 (chlorure de méthylène - méthanol ammoniacal 10% - 95/5 v/v)
- Analyse (C₁₉H₂₇NO₂) C, H, N, O
- IR (KBr) : 2900, 2800, 1450, 1290, 1060, 940, 760 cm⁻¹
- RMN : 0,1-0,2(m,2H) ; 0,3-0,6(m,2H) ; 0,6-1,0(m,1H) ; 1,0-2,6(m,9H) ; 2,3(s,3H) ; 2,6-2,9(m,2H) ; 3,9-4,2(m,4H) ; 6,9-7,5(m,4H)

### EXEMPLE 13 : 2-[2-[(N-cyclobutylméthyl-N-méthyl)amino]éthyl]-1-(N-hydroxy)imino-1,2,3,4-tétrahydronaphtalène.

(Formule I.b; V₁-V₂=N-OH;X=Y=H; R₁=CH₃; A=CH₂; m=1; n=3)

Dans un ballon on introduit 0,90g (3,3 mmole) de 2-[2-[(N-cyclobutylméthyl-N-méthyl)amino]éthyl]-1-oxo-1,2,3,4-tétrahydronaphtalène (exemple 3 - stade 3), 0,69 g (10,0 mmole) de chlorhydrate d'hydroxylamine, 50 ml de pyridine anhydre et 5,0 ml d'éthanol absolu.

Le mélange est agité et chauffé. La solution obtenue est maintenue au reflux durant 2 heures puis les solvants éliminés par distillation sous vide.

Le résidu est repris par 50 ml d'eau. Le mélange alcalinisé à pH 12 par addition de NaOH 10 N. Le précipité obtenu est filtré, lavé à l'éther puis séché à 50° C sous vide. Poids 0,30 g. Rdt = 31,8% F = 120-122°C
- CCM : Rf = 0,50 (chlorure de méthylène - méthanol ammoniacal 10% - 95/5 v/v)
- Analyse (C₁₈H₂₆N₂O) C, H, N, O
- IR (KBr) : 2900, 1490, 1460, 1440, 1160, 1060, 1000, 960, 760 cm⁻¹
- RMN : 1,5-3,7(m,19H) ; 2,2(s,3H) ; 7,0-8,1(m,4H)

### EXEMPLE 14 : Chlorhydrate de 3-[2-[(N-cyclopropylméthyl-N-méthyl)amino]éthyl]-4-chromanone.

(Formule I.a; X=Y=H; R₁=CH₃; A=O; m=1; n=2)

Le produit est préparé selon le mode opératoire de l'exemple 2-stades 1, 2 et 3 à partir de l'acide 4-chromanone-2-acétique et de N-cyclopropylméthyl-méthylamine.

### Stade 1 : N-cyclopropylméthyl-N-méthyl-2-[(4-chromanone)-3-yl]-acétamide. (III; X=Y=H; R₁=CH₃; A=O; m=1; n=2) Rdt = 39,1%

- CCM : Rf = 0,30-0,40 (acétate d'éthyle-hexane 70-30 v/v)

### Stade 2 : 3-[2-[(N-cyclopropylméthyl-N-méthyl)amino]éthyl]-4-chromanol. (isomères II; X=Y=H; R₁=CH₃; A=O; m=1; n=2) Rdt = 98,2%

- CCM : Rf = 0,40-0,50 (chlorure de méthylène-méthanol ammoniacal 10% 95-5 v/v)

### Stade 3 : 3-[2-[(N-cyclopropylméthyl-N-méthyl)amino]éthyl]-4-chromanone. (I.a; X=Y=H; R₁=CH₃; A=O; m=1; n=2) Rdt = 68,0%

- CCM : Rf = 0,40-0,50 (chlorure de méthylène - méthanol ammoniacal 10% - 95/5 v/v)

### chlorhydrate

Rdt = 77,1% F = 170-172°C
- CCM : Rf = 0,45 chlorure de méthylène
- Analyse (C₁₆H₂₂ClNO₂) C, H, Cl, N, O
- IR (KBr) : 2950, 2400, 1680, 1600, 1480, 1300, 1220, 1030, 820, 740 cm⁻¹
- RMN : 0,05-0,4(m,2H) ; 0,4-0,6(m,2H) ; 0,65-1,0(m,1H) ; 1,4-1,8(m,1H) ; 1,9-3,0(m,6H) ; 2,35(s,3H) ; 4,1-4,7(m,2H) ; 6,85-7,95(m,4H)

### EXEMPLE 15 : Chlorhydrate de 3-[2-[(N-cyclohexylméthyl-N-méthyl)amino]éthyl]-4-chromanone.

(Formule I.a; X=Y=H; R₁=CH₃; A=O; m=1; n=5)

### Stade 1 : N-cyclohexylméthyl-N-méthyl-2-[(4-chromanone)-3-yl]-acétamide. (III; X=Y=H; R₁=CH₃; A=O; m=1; n=5)

Dans un réacteur protégé de l'humidité on introduit 250ml de chlorure de méthylène déshydraté sur tamis moléculaire puis on dissout 15,05g (73 mmole) d'acide 4-chromanone-2-acétique et 11,55g (90 mmole) de chlorhydrate de N-cyclohexylméthylméthylamine. Sous agitation et à 25°C on ajoute en solution dans 100ml de chlorure de méthylène 20,85g (109 mmole) de chlorhydrate de [(diméthylamino-3)-propyl]-1-éthyl-3-carbodiimide. La solution est maintenue 2 heures sous agitation à 20-25°C puis extraite successivement par 100ml de solution HCl N, 2 fois 100ml d'eau puis 100ml de solution saturée en NaHCO₃ et enfin 2 fois 100ml d'eau.

Après déshydratation sur Na₂SO₄ le chlorure de méthylène est évaporé par distillation sous vide. Le résidu brut est une huile jaune (22,15g) qui est purifiée par chromatographie sur colonne de silice. L'élution par un mélange acétone-acétate d'éthyle 70-30 v/v permet d'obtenir le produit pur.
Poids = 18,85 g Rdt = 85,1%
- CCM : Rf = 0,90-0,95 (Acétate d'éthyle)

### Stade 2 : 3-[2-[(N-cyclohexylméthyl-N-méthyl)amino]éthyl]-4-chromanol. (isomères II; X=Y=H; R₁=CH₃; A=O; m=1; n=5)

L'intermédiaire est obtenu par réduction au LAH de l'amide préparé au stade précédent et tel que décrit à l'exemple 2 - stade 2. Rdt = 88,8%
- CCM : Rf = 0,75-0,90 (Chlorure de méthylène-méthanol ammoniacal à 10% 90-10 v/v)

### Stade 3 : 3-[2-[(N-cyclohexylméthyl-N-méthyl)amino]éthyl]-4-chromanone. (I.a; X=Y=H; R₁=CH₃; A=O; m=1; n=5)

Obtenu par oxydation du composé du stade précédent selon la méthode décrite à l'exemple 2 - stade 3. Rdt = 65,8%
- CCM : Rf = 0,90 (Chlorure de méthylène-méthanol ammoniacal à 10% 90-10 v/v)

### chlorhydrate

Rdt = 59% F = 204-205°C
- CCM : Rf = 0,85 (Chlorure de méthylène-méthanol ammoniacal à 10% 90-10 v/v)
- Analyse (C₁₉H₂₈ClNO₂) C, H, Cl, N, O
- IR (KBr) : 2900,2850,2600,1680,1600,1480,1290,1120,930,750cm⁻¹
- RMN : 0,5-2,65 (m,17H);2,15(s,3H);2,65-3,05(m,1H);
   4,1-4,7(m,2H);6,7-7,1(m,2H);7,25-7,55(m,1H);7,7-7,95 (m,1H)

### EXEMPLE 16 : chlorhydrate de 3-[2-[(N-cyclohexylméthyl-N-méthyl)amino]éthyl]-6-fluooro-4-chromanone.

(Formule I.a; X=F; Y=H; R₁=CH₃; A=O; m=1; n=5)

### Stade 1 : N-cyclohexylméthyl-N-méthyl-2-[(6-fluoro-4-chromanone)-3-yl]-acétamide. (III; X=F; Y=H; R₁=CH₃; A=O; m=1; n=5)

Le composé intermédiaire est préparé par condensation de l'acide 6-fluoro-4-chromanone-2-acétique avec la N-cyclohexylméthyl-méthylamine tel que décrit à l'exemple 15 -stade 1 qui précède. Rdt = 95%
- CCM : Rf = 0,60 (Acétate d'éthyle-hexane 70-30 v/v)

### Stade 2 : 3-[2-[(N-cyclohexylméthyl-N-méthyl)amino]éthyl]-6-fluoro-4-chromanol. (isomères II; X=F; Y=H; R₁=CH₃; A=O; m=1; n=5)

Préparé par réduction avec LAH comme décrit à l'exemple 2 - stade 2. Rdt = 67%
- CCM : Rf = 0,70-0,80 (Chlorure de méthylène-méthanol ammoniacal à 10% 95-5 v/v)

### Stade 3 : 3-[2-[(N-cyclohexylméthyl-N-méthyl)amino]éthyl]-6-fluoro-4-chromanone. (I.a; X=F; Y=H; R₁=CH₃; A=O; m=1; n=5)

Préparé selon l'exemple 2 - stade 3. Rdt = 64%
- CCM : Rf = 0,90 (Chlorure de méthylène-méthanol ammoniacal à 10% 95-5 v/v)

### chlorhydrate

Rdt = 80% F = 228-230°C
- CCM : Rf = 0,80-0,85 (Chlorure de méthylène-méthanol ammoniacal à 10% 95-5 v/v)
- Analyse (C₁₉H₂₇ClFNO₂) C, H, Cl, F, N, O
- IR (KBr) : 2800,2750,2600,1685,1620,1490,1430,1280,820,740cm⁻¹
- RMN : 0,4-2,6(m,17 H); 2,15(s,3H);2,6-3,05(m,1H);4,1-4,7(m,2H);6,8-7,3(m,2H) ;7,4-7,65(m,1H)

### EXEMPLE 17 : Chlorhydrate de 2-[2-[(N-allyl-N-méthyl)amino]éthyl]-1-oxo-1,2,3,4-tétrahydronaphtalène.

(Formule I.a; X=Y=H; A=CH₂; R₁=CH₃; m=1; n=1)

Selon le mode opératoire décrit à l'exemple 2, à partir d'acide 1-oxo-1,2,3,4-tétrahydro-2-naphtalène acétique et de N-allylméthylamine on obtient successivement aux stades 1 à 3 les composés suivants.

### Stade 1 : N-allyl-N-méthyl-2-[2-(1-oxo-1,2,3,4-tétrahydronaphtyl)]-acétamide. (III; X=Y=H; A=CH₂; R₁=CH₃; m=1; n=1) Rdt = 75%

- CCM : Rf = 0,70-0,80 (acétate d'éthyle)

### Stade 2 : 2-[2-[(N-allyl-N-méthyl)amino]éthyl]-1-hydroxy-1,2,3,4-tétrahydronaphtalène. (isomères II; X=Y=H; A=CH₂; R₁=CH₃; m=1; n=1) Rdt = 83%

- CCM :Rf = 0,50-0,60-0,70 (chlorure de méthylène-méthanol,NH₄OH 10%-95/5-v/v)

### Stade 3 : 2-[2-[(N-allyl-N-méthyl)amino]éthyl]-1-oxo-1,2,3,4-tétrahydronaphtalène. (I.a; X=Y=H; A=CH₂; R₁=CH₃; m=1; n=1) Rdt = 74% (huile violette)

- CCM : Rf= 0,40-0,50 (chlorure de méthylène -méthanol, NH4OH 10%-95/5-v/v)

### Chlorhydrate

Rdt = 57,9% F = 132-134°C
- CCM : Rf = 0,40-0,60 (chlorure de méthylène -méthanol, NH4OH 10%-95/5-v/v)
- Analyse (C₁₆H₂₂ClNO) C, H, Cl, N, O
- IR (KBr) : 2900, 2600, 2500, 1680, 1600, 1440, 1305, 1290, 1220, 1100, 740 cm⁻¹
- RMN : 1,4-2,7(m,7H) ; 2,25(s,3H) ; 2,85-3,1(m,4H); 5,0-5,3 (m,2H) ; 5,65-6,1 (m,1H) ; 7,1-7,55 (m,3H) ; 7,90-8,1 (m,1H)

Les composés de l'invention (I) et leurs sels ont montré leur capacité d'interaction avec les récepteurs sigma dans les essais de screening biochimiques et pharmacologiques réalisés "in vitro" avec les ligands (+) [³H]SKF10,047 et [³H]DTG, ligands qui mettent en évidence les affinités de liaison aux récepteurs sigma des composés étudiés. Des tests de binding aux récepteurs de la phencyclidine et de la dopamine D₂ ont été réalisés pour étudier les interactions éventuelles et indésirables des composés de l'invention avec ces récepteurs.

Les ligands utilisés sont le [³H]TCP pour la phencyclidine et le [³H]-Spipérone pour la dopamine.

Par ailleurs des tests "in vivo" ont permis de mettre en évidence la capacité des composés de l'invention (I) d'inhiber les convulsions induites par des électrochocs chez le rat et également leur capacité à inhiber des ulcères gastroduodénaux provoqués par administration de cystéamine.

### 1) Etude "in vitro"

Les expériences de binding sont réalisées avec les ligands sigma (+)[³H]SKF10,047 et [³H]TCP, selon la technique décrite par Largent B.L. et al. dans J.Pharmacol.Exp.Thér.238, 1986, p 739-748 dont le principe est de mettre en compétition les affinités respectives du produit à étudier et celle d'un ligand radioactif caractéristique des récepteurs sigma.

Le binding avec le ligand [³H]DTG est réalisé selon la technique de Weber, E.M. et al., 1986, Proc.Natl.Acad.Sci., 83:8784-8788, et le binding avec le ligand [³H]-Spipérone est réalisé selon la technique de Fields,J.Z, Reisine,R.D. and Yamamura, H.I., Brain Res., 136,578(1977).

La technique consiste à faire incuber des solutions de concentrations appropriées des produits à l'essai avec des prélèvements standards de membranes chargées par le ligand marqué puis à déterminer après filtration la radioactivité de la solution.

Les résultats sont traités de façon à calculer la CI₅₀ du produit à l'étude, qui représente la concentration nanomolaire de solution capable d'inhiber 50% des liaisons du ligand tritié aux récepteurs sigma des membranes utilisées. Ils sont présentés aux tableaux 1.A et 1.B qui suivent en comparaison avec les résultats obtenus avec l'halopéridol choisi comme composé de référence.

Les tests de binding avec SKF 10,047, sont effectués avec des membranes de cerveaux de cobayes ou de rats, ceux avec DTG et TCP sont effectués avec des membranes de cerveaux de cobayes; les tests de binding D₂ sont effectués avec des membranes de cerveaux de rats.

**Tableau 1.A**

| TESTS COMPARATIFS DE BINDING IN VITRO | | | | |
|---|---|---|---|---|
| PRODUITS TESTES | SKF 10,047 (cobaye) CI₅₀ nM | DTG (cobaye) CI₅₀ nM | PCP (cobaye) CI₅₀ nM | D₂ (rat) CI₅₀ nM |
| EXEMPLE 2 | 12 | 52,00 | >10000 | >10000 |
| EXEMPLE 3 | 5,24 | n.t | >10000 | 1172 |
| EXEMPLE 4 | 2,83 | 56,00 | >10000 | 1910 |
| Halopéridol | 8,95 | 22,67 | 1268 | 2 |
| n.t : non testé. | | | | |

**Tableau 1.B**

| RESULTATS DE BINDING SIGMA (SKF 10,047/membranes cerveaux de rats) | | | |
|---|---|---|---|
| PRODUITS TESTES | CI₅₀ nM | PRODUITS TESTES | CI₅₀ nM |
| EXEMPLE 1 | 10,7 | EXEMPLE 11 | 6,6 |
| EXEMPLE 2 | 19,4 | EXEMPLE 12 | 30,5 |
| EXEMPLE 3 | 11,1 | EXEMPLE 13 | 7,7 |
| EXEMPLE 4 | 10,6 | EXEMPLE 14 | 13,7 |
| EXEMPLE 5 | 8,2 | EXEMPLE 15 | 5,7 |
| EXEMPLE 6 | 8,0 | EXEMPLE 16 | 6,2 |
| EXEMPLE 9 | 10,4 | EXEMPLE 17 | 13,3 |
| EXEMPLE 10 | 5,7 | HALOPERIDOL | 24,6 |

Ces résultats montrent que les produits de l'invention (I) illustrée par les composés des exemples précédents, ont une spécificité d'affinité manifeste pour les récepteurs sigma d'intensité supérieure à celle montrée par l'Halopéridol. De plus, et de façon remarquable en comparaison avec ce produit de référence, les composés de l'invention (I) montrent, comme il est reporté au tableau 1.A, une affinité que l'on peut considérer nulle pour les récepteurs PCP et une affinité également nulle sinon négligeable pour les récepteurs D₂ ce qui est démonstratif de leur intérêt thérapeutique.

### 2) Etude "in vivo"

### a) Convulsions chez le rat : électrochoc

Les propriétés psychotropes des composés (I) ont été déterminées par la protection des convulsions induites par électrochocs chez le rat.

L'émission d'un électrochoc provoque chez l'animal un état convulsif caractérisé par l'extension convulsive des pattes avant et arrière.

Pratiquement l'étude est réalisée sur des lots de 10 rats mâles Sprague Dawley, d'un poids de 100 grammes environ auxquels on administre le produit à étudier en solution aqueuse par voie sous-cutanée à raison de 0,5 ml par 100 grammes de poids de l'animal.

On provoque ensuite un choc électrique 30 minutes après l'injection grâce à un appareil pour électrochoc ECT UNIT 7801 UGO BASILE (APELEX) : fréquence de 50cps/sec, largeur du choc de 0,6ms, durée du choc 1sec, intensité 90mA. Dans ces conditions expérimentales, les animaux présentent des convulsions toniques qui se manifestent par une extension des pattes avant et arrières. Chaque animal reçoit alors un score qui est égal à 1 pour la présence de convulsions toniques , et zéro pour l'absence de convulsions toniques. Dans chaque lot, le pourcentage d'animaux ne présentant pas de convulsions est calculé. Les résultats sont comparés de façon statistique entre lot témoin et lot traité par le test de Fisher R.A et Yates F. (Biometrika, 1948, 35-149) et la DE₅₀ (dose du composé testé entrainant une protection chez 50% des animaux) est calculée par la méthode de Litchfield J.T et Wilcoxon F. (J. Pharmacol. Exp.Therap., 1949, 96-99).

Les résultats de l'étude sont reportés pour les produits de l'invention dans le tableau 2.

### b) Ulcère à la cystéamine

L'activité des composés de l'invention sur le tractus gastrointestinal a été montré chez le rat par leur aptitude à inhiber les ulcères gastroduodénaux provoqués par administration de cystéamine. (Robert et coll.,Digestion, 1974, 11, 199-211).

Pratiquement, l'étude est réalisée d'après la méthode décrite par Selye,H. and Szabo,S., Nature, 1973, 244:458-459 sur des lots de rats mâles Sprague Dawley d'un poids moyen de 200 g auquels on administre par injection sous-cutanée une solution de chlorhydrate de cystéamine à raison de 400 mg/kg, les produits à tester étant administrés aux animaux par voie orale ou intraduodénale ou bien par voie intrapéritonéale 1 heure trente avant l'agent ulcérogène. Dix huit heures après, les rats sont sacrifiés par élongation, leur estomac et duodénum sont prélevés, rincés avec du soluté physiologique et épinglés sur un carton. La présence d'ulcères de la zone antro-pylorduodénale est recherchée et leur surface, exprimée en mm², est évaluée en multipliant les deux axes perpendiculaires principaux de la lésion. L'analyse statistique des résultats est réalisée à partir du test de Student pour les surfaces ulcérées comparativement à un lot témoin. Les résultats sont présentés au tableau 2 et exprimés en DE₅₀ de scores d'ulcération qui sont les doses efficaces en (mg/kg) de produit pour inhiber 50% des ulcères provoqués par la cystéamine.

**Tableau 2**

| TESTS IN VIVO | | |
|---|---|---|
| PRODUITS TESTES | Ulcère cystéamine DE₅₀ mg/kg | Electrochocs DE₅₀ mg/kg |
| EXEMPLE 2 | n.t | 0,1 |
| EXEMPLE 3 | 8,6 (po) 5,7 (ip) | 0,9 |
| EXEMPLE 4 | 15,6 (po) | 1,0 |
| EXEMPLE 10 | n.t | 1,6 |
| EXEMPLE 12 | 11,6 (ip) | 1,7 |
| EXEMPLE 14 | n.t | 3,5 |
| (po) = voie orale (ip) = voie intrapéritonéale (n.t) = non testé | | |

La toxicité aigüe des produits de l'invention a été recherchée après administration par voie orale chez le rat ce qui a permis de déterminer une valeur approchée de leur DL₅₀, qui est la dose léthale provoquant la mort de 50% des animaux dans les conditions de l'expérience. A des doses près de cent fois supérieures à leur dose physiologiquement active, cette toxicité a été considérée comme négligeable.

Telles que décrites, ces propriétés pharmacologiques associées à la faible toxicité des composés de l'invention permettent d'envisager leur utilité sous forme de médicaments pour des traitements préventifs et curatifs d'affections entrainant certains désordres psychiques, notamment les états psychotiques comme les états dépressifs, les troubles de la mémoire et du comportement, le stress et l'anxiété, ainsi que dans le cas de dysfonctionnements du tractus gastrointestinal comme par exemple divers ulcères dont ceux liés au stress.

De façon courante les posologies sont comprises de 1 à 1000mg et plus particulièrement de 5 à 500mg de produit selon la nature et la gravité de l'affection à traiter. Ces doses thérapeutiques journalières peuvent être réparties en plusieurs prises. De façon générale une posologie journalière de 5 mg à 500 mg de produit répartis en deux à quatre prises conduit à un résultat thérapeutique satisfaisant.

L'administration des produits de l'invention aux patients à traiter est réalisée sous la forme de médicaments de nature adaptée à l'affection à soigner.

Selon les cas, les préparations médicamenteuses seront, comme exemples non limitatifs, des comprimés, dragées, capsules, poudres, solutions, suspensions, gels ou suppositoires. Ces diverses formes pharmaceutiques sont préparées à partir des produits sous forme de base ou de leurs sels et selon des méthodes couramment mises en oeuvres dans la pratique pharmaceutique.

Généralement dans les formes médicamenteuses de nature "solide", le principe actif représente de 2 à 50 % en poids du total de la forme terminée alors que les excipients représentent de 98 à 50 %. Pour les formes "liquides", ou pouvant être considérées comme telles, la quantité de principe actif est comprise entre 0,1 et 10 % en poids de la forme terminée alors que les excipients peuvent représenter de 99,9 à 90 % en poids de cette forme.

A titre d'illustration il est décrit la formule et la préparation de comprimés et de soluté isotonique avec le composé de l'exemple 2.

### Comprimés

### Formule :

| | |
|---|---|
| - principe actif (composé de l'exemple 2) | 10,0 à 50,0 mg |
| - polyvinylpyrrolidone | 20,0 mg |
| - carboxyméthylamidon | 8,0 mg |
| - stéarate de magnésium | 2,0 mg |
| - silice colloïdale | 0,4 mg |
| - lactose en quantité suffisante pour | 200,0 mg |

### Préparation :

Le principe actif en solution hydroalcoolique est mélangé au lactose puis granulé avec la polyvinylpyrrolidone également en solution. Les grains sont séchés et tamisés sur une grille d'ouverture de 1 mm. Le carboxyméthylamidon est mélangé à la silice colloïdale puis ajouté aux granulés. On mélange ensuite intimement avec le stéarate de magnésium puis comprime à raison de 200,0 mg par comprimé.

### Soluté isotonique injectable

### Formule :

| | |
|---|---|
| - substance active (I), chlorhydrate de l'exemple 2 | 10,0 mg |
| - chlorure de sodium | 9,0 mg |
| - eau distillée en quantité suffisante pour | 1,0 ml |

### Préparation :

Le soluté isotonique est réparti en ampoules de volume approprié qui sont, après scellement, stérilisées par des moyens thermiques habituels ou bien le soluté est stérilisé par filtration, réparti en ampoules qui sont ensuite scellées, l'ensemble de ces opérations étant réalisé sous atmophère stérile.

## Revendications

1. Nouvelles cycloalkylalkylamines ligands aux récepteurs sigma de formule générale (I) 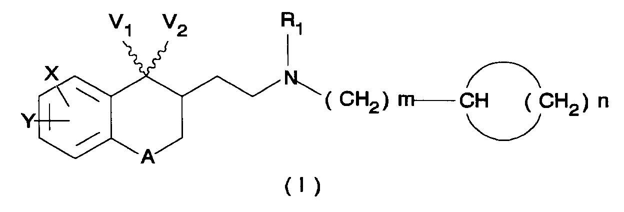 dans laquelle :
R₁ est H ou alkyl de C₁ à C₄ ;
X et Y identiques ou différents et sont H, OH, alkyl de C₁ à C₄, alkoxy de C₁ à C₄, halogène, nitrile ou cyanogène;
V₁ et V₂ forment ensemble une double liaison liée à un atome d'oxygène ou bien à un radical hydroxyimino N-OH, ou bien sont reliés en une chaîne éthylène dioxy -O-CH₂-CH₂-O-;
A représente une liaison de valence, un atome d'oxygène, un groupe méthylène ou encore un groupe éthylène;
m est égal à 0, 1 ou 2;
n a pour valeur un entier de 1 à 5;
et leurs sels d'addition aux acides pharmaceutiquement acceptables.

2. Composés selon la revendication 1 caractérisés en ce que V₁ et V₂ forment ensemble une double liaison liée à un atome d'oxygène.

3. Composés selon l'une des revendications 1 ou 2 caractérisés en ce que A représente un groupe méthylène.

4. Composés selon l'une des revendications 1 à 3 caractérisés en ce que R₁ est CH₃.

5. Composés selon l'une des revendications 1 à 4 caractérisés en ce que m = 1 ou 2 et n = 2 ou 3.

6. Cycloalkylalkylamines (I) selon la revendication 1 qui sont :
- le 2-[2-[(N-cyclobutylméthyl-N-méthyl)amino]éthyl]-1-oxo-1,2,3,4-tétrahydronaphtalène et son chlorhydrate,
- le 2-[-2(N-cyclopropyléthyl-N-méthyl)amino]éthyl-1-oxo-1,2,3,4-tétrahydronaphtalène et son chlorhydrate.

7. Procédé de préparation des cycloalkylalkylamines (I) définies à la revendication 1, caractérisé en ce qu'il consiste
- pour préparer une cycloalkylalkylamine dans laquelle V₁ et V₂ forment ensemble une double liaison liée à un atome d'oxygène et qui correspond à une cycloalkylalkylamine de formule (I.a) 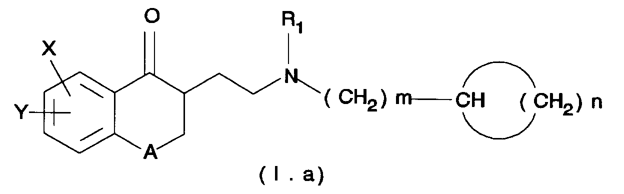
soit à cycliser et décarboxyler par chauffage en milieu acide un intermédiaire malonique (VIII) 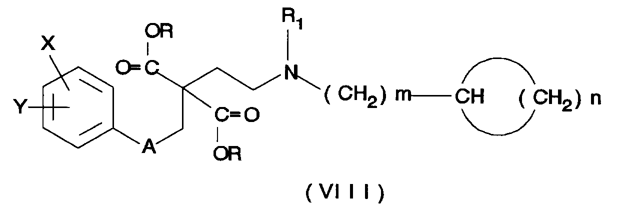 dans lequel R est l'hydrogène, alkyl de C₁ à C₄ ou cycloalkyl inférieur,
soit à cycliser un intermédiaire acide (IX) 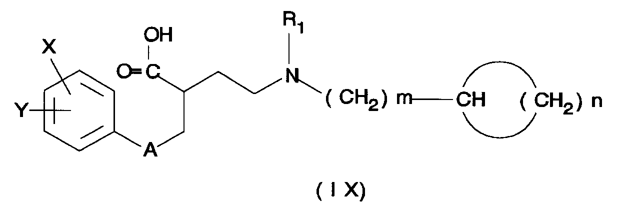 par une méthode d'acylation de type Friedel-Crafts,
soit à oxyder la fonction hydroxyle d'un amino alcool (II)

8. Médicament, utile au traitement des affections induites par un dysfonctionnement des mécanismes physiologiques faisant intervenir les récepteurs sigma chez les mammifères ou chez l'homme caractérisé en ce qu'il comprend une cyloalkylalkylamine (I) suivant l'une des revendications 1 à 6, et un excipient thérapeutiquement acceptable.

9. Amino-alcools intermédiaires de formule (II) 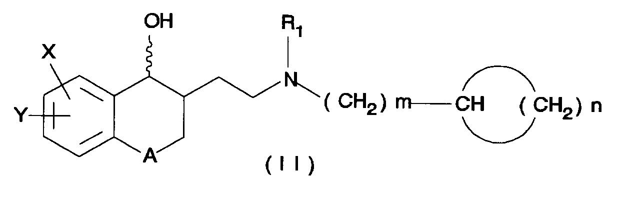 dans laquelle,
R₁ est H ou alkyl de C₁ à C₄;
X et Y identiques ou différents sont H, OH, alkyl de C₁ à C₄, alkoxy de C₁ à C₄, halogène ou nitrile;
A représente une liaison de valence, un atome d'oxygène, un groupe méthylène ou encore un groupe éthylène;
m est égal à 0, 1 ou 2;
n a pour valeur un entier de 1 à 5.

## Claims

1. New cycloalkylalkylamines which are sigma-receptor ligands of general formula (I) 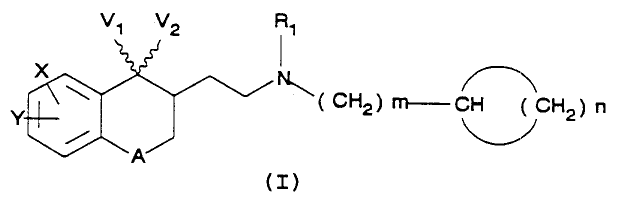 in which:
R₁ is H or C₁ to C₄ alkyl;
X and Y, which may be identical or different, are H, OH,
C₁ to C₄ alkyl, C₁ to C₄ alkoxy, halogen, nitrile or cyanogen;
V₁ and V₂ together form a double bond attached to an oxygen atom or else to a hydroxyimino radical N-OH, or else are linked as an ethylenedioxy chain -O-CH₂-CH₂-O-;
A represents a valency bond, an oxygen atom, a methylene group or alternatively an ethylene group;
m is equal to 0, 1 or 2;
n has the value of an integer from 1 to 5;
and their addition salts with pharmaceutically acceptable acids.

2. Compounds according to Claim 1, characterized in that V₁ and V₂ together form a double bond attached to an oxygen atom.

3. Compounds according to one of Claims 1 and 2, characterized in that A represents a methylene group.

4. Compounds according to one of Claims 1 to 3, characterized in that R₁ is CH₃.

5. Compounds according to one of Claims 1 to 4, characterized in that m = 1 or 2, and n = 2 or 3.

6. Cycloalkylalkylamines (I) according to Claim 1, which are:
- 2-[2-[(N-cyclobutylmethyl-N-methyl)amino]ethyl]-1-oxo-1,2,3,4-tetrahydronaphthalene and its hydrochloride,
- 2-[2-[(N-cyclopropylethyl-N-methyl)amino]ethyl]-1-oxo-1,2,3,4-tetrahydronaphthalene and its hydrochloride.

7. Process for preparing the cycloalkylalkylamines (I) defined in Claim 1, characterized in that it consists
- for preparing a cycloalkylalkylamine in which V₁ and V₂ together form a double bond attached to an oxygen atom, and which corresponds to a cycloalkylalkylamine of formula (I.a) 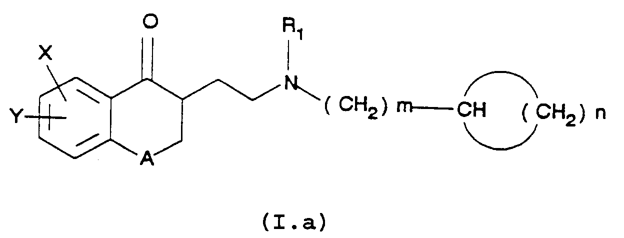
either in cyclizing and decarboxylating by heating in an acid medium a malonic intermediate (VIII) 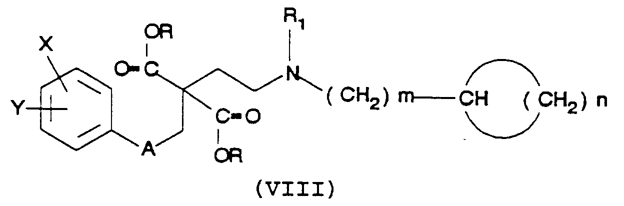 in which R is hydrogen, C₁ to C₄ alkyl or lower cycloalkyl,
or in cyclizing an acid intermediate (IX) 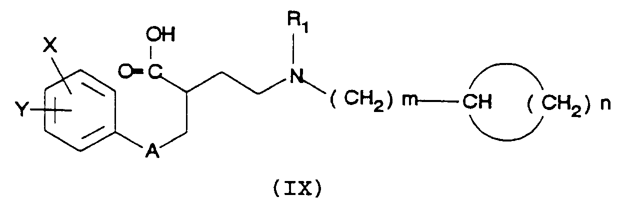 by a Friedel-Crafts type acylation method,
or in oxidizing the hydroxyl function of an amino alcohol (II) 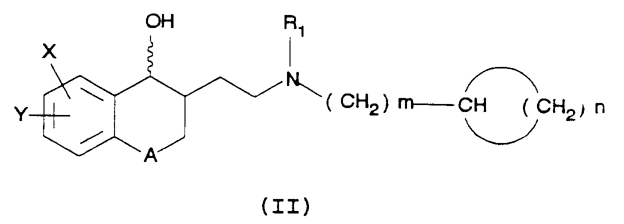 with an oxidizing reagent derived from chromium and,
- for preparing a cycloalkylalkyamine in which V₁ and V₂ form a double bond attached to a hydroxyimino radical, corresponding to the formula (I.b) 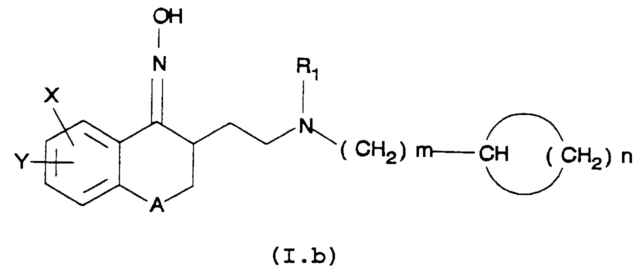
in reacting a cycloalkylalkylamine (I.a), already shown, with hydroxylamine and,
- for preparing a cycloalkylalkylamine in which V₁ and V₂ are linked as an ethylenedioxy chain, corresponding to the formula (I.c) 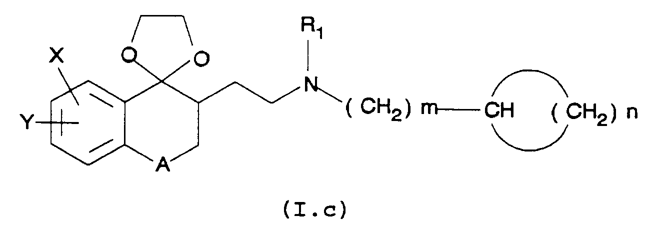
in reacting a cycloalkylalkylamine (I.a), already shown, by heating with ethylene glycol in a acid medium, or in reducing an amide intermediate of formula (III.a) 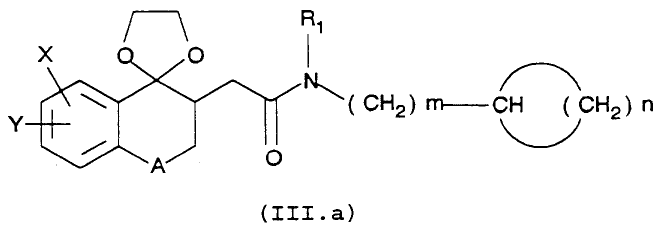 with a metal hydride or organometallic hydride.

8. Medicinal product which is useful in the treatment of conditions induced by a dysfunction of the physiological mechanisms involving the sigma receptors in mammals or in man, characterized in that it comprises a cycloalkylalkylamine (I) according to one of Claims 1 to 6 and a therapeutically acceptable excipient.

9. Intermediate amino alcohols of formula (II) 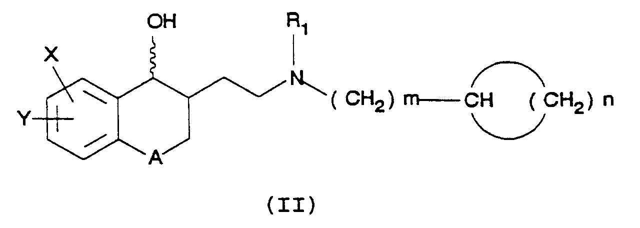 in which
R₁ is H or C₁ to C₄ alkyl;
X and Y, which may be identical or different, are
H, OH, C₁ to C₄ alkyl, C₁ to C₄ alkoxy, halogen or nitrile;
A represents a valency bond, an oxygen atom, a methylene group or alternatively an ethylene group;
m is equal to 0, 1 or 2;
n has the value of an integer from 1 to 5.

## Patentansprüche

1. Neue Cycloalkylalkylaminliganden für Sigmarezeptoren der allgemeinen Formel (I) 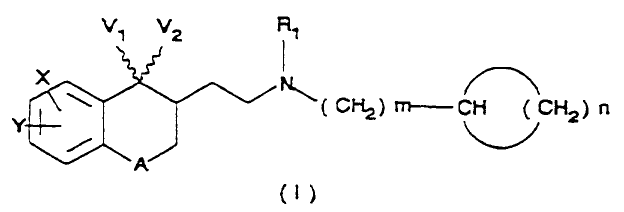 worin
R₁ H oder C₁- bis C₄-Alkyl,
X und Y, gleich oder verschieden, H, OH, C₁- bis C₄-Alkyl,
C₁- bis C₄-Alkoxy, Halogen, Nitril oder Cyan bedeuten,
V₁ und V₂ zusammen eine mit einem Sauerstoffatom oder auch mit einem Hydroxyiminorest N-OH verknüpfte Doppelbindung bilden oder auch an eine Ethylendioxykette -O-CH₂-CH₂-O-gebunden sind,
A für eine Valenzbindung, ein Sauerstoffatom, eine Methylengruppe oder auch eine Ethylengruppe steht,
m gleich 0, 1 oder 2 ist und
n eine ganze Zahl im Wert von 1 bis 5 bedeutet,
und deren pharmazeutisch verträglichen Additionssalze.

2. Verbindungen nach Anspruch 1, dadurch gekennzeichnet, daß V₁ und V₂ zusammen eine mit einem Sauerstoffatom verknüpfte Doppelbindung bilden.

3. Verbindungen nach Anspruch 1 oder 2, dadurch gekennzeichnet, daß A für eine Methylengruppe steht.

4. Verbindungen nach einem der Ansprüche 1 bis 3, dadurch gekennzeichnet, daß R₁ CH₃ bedeutet.

5. Verbindungen nach einem der Ansprüche 1 bis 4, dadurch gekennzeichnet, daß m = 1 oder 2 und n = 2 oder 3 ist.

6. Cycloalkylalkylamine (I) nach Anspruch 1, bei denen es sich um folgende handelt:
- 2-[2-[(N-Cyclobutylmethyl-N-methyl)amino]ethyl]-1-oxo-1,2,3,4-tetrahydronaphthalin und dessen Hydrochlorid,
- 2-[2-[(N-Cyclopropylethyl-N-methyl)amino]ethyl]-1-oxo-1,2,3,4-tetrahydronaphthalin und dessen Hydrochlorid.

7. Verfahren zur Herstellung von Cycloalkylalkylaminen (I) nach Anspruch 1, dadurch gekennzeichnet, daß man
- zur Herstellung eines Cycloalkylalkylamins, worin V₁ und V₂ zusammen eine mit einem Sauerstoffatom verknüpfte Doppelbindung bilden und das einem Cycloalkylalkylamin der Formel (I.a) entspricht, 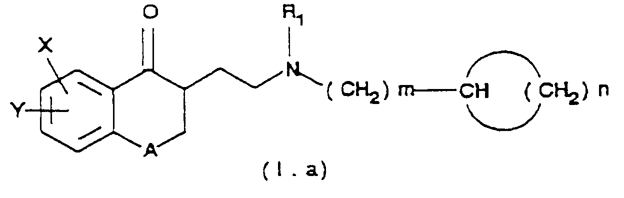
entweder eine malonsäurederivathaltige Zwischenstufe (VIII) 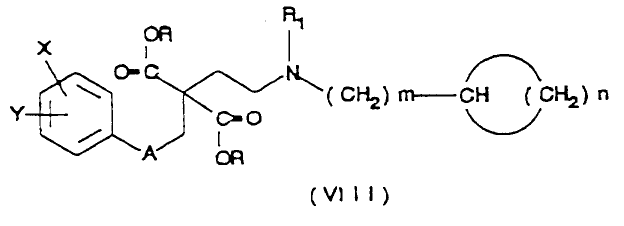 worin R Wasserstoff, C₁- bis C₄-Alkyl oder Nieder-Cycloalkyl bedeutet,
durch Erhitzen in saurem Medium cyclisiert und decarboxyliert, oder eine säurehaltige Zwischenstufe (IX) 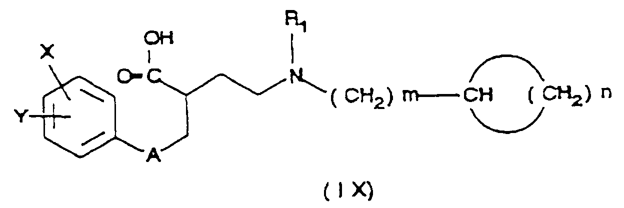 nach einem Acylierungsverfahren vom Typ Friedel-Crafts cyclisiert,
oder die Hydroxylfunktion eines Aminoalkohols (II) 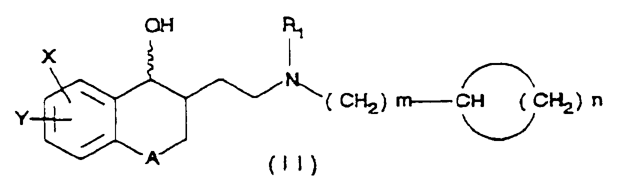 mit einem von Chrom abgeleiteten Oxidationsmittel oxidiert und
- zur Herstellung eines Cycloalkylalkylamins, worin V₁ und V₂ zusammen eine mit einem Hydroxyiminorest verknüpfte Doppelbindung bilden, entsprechend der Formel (I.b) 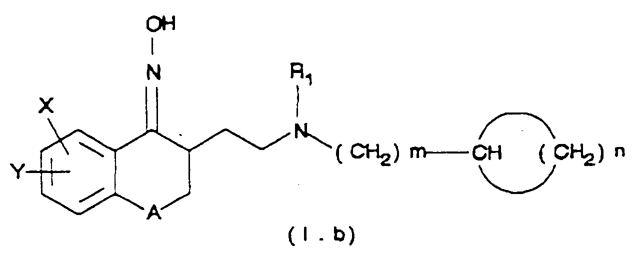
ein bereits genanntes Cycloalkylalkylamin (I.a) mit Hydroxylamin umsetzt und
- zur Herstellung eines Cycloalkylalkylamins, worin V₁ und V₂ an eine Ethylendioxykette gebunden sind, entsprechend der Formel (I.c) 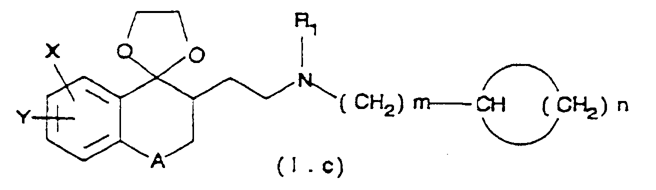
ein bereits genanntes Cycloalkylalkylamin (I.a) durch Erhitzen in saurem Medium mit Ethylenglykol umsetzt oder mit einem Metallhydrid oder einem metallorganischen Hydrid zu einer Amidzwischenstufe der Formel (III.a) 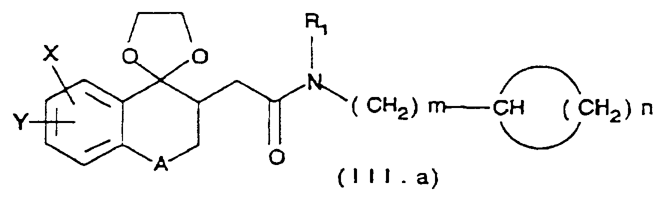 reduziert.

8. Arzneimittel, das sich für die Behandlung von Krankheitszuständen eignet, die durch eine Funktionsstörung der physiologischen Vorgänge hervorgerufen werden, unter Beteiligung der Sigmarezeptoren bei Säugetieren oder beim Menschen, dadurch gekennzeichnet, daß es ein Cycloalkylalkylamin (I) nach einem der Ansprüche 1 bis 6 und einen therapeutisch annehmbaren Träger enthält.

9. Aminoalkoholzwischenstufen der Formel (II) 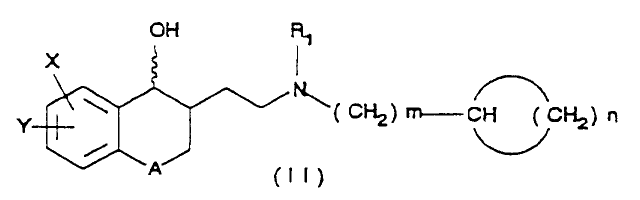 worin
R₁ H oder C₁- bis C₄-Alkyl,
X und Y, gleich oder verschieden, H, OH, C₁- bis C₄-Alkyl,
C₁- bis C₄-Alkoxy, Halogen oder Nitril bedeuten,
A für eine Valenzbindung, ein Sauerstoffatom, eine Methylengruppe oder auch eine Ethylengruppe steht,
m gleich 0, 1 oder 2 ist und
n eine ganze Zahl im Wert von 1 bis 5 bedeutet.
